# FASCICULE DE BREVET EUROPEEN

(11) **EP 2 524 030 B1**
(45) Date de publication et mention de la délivrance du brevet: **10.08.2016**
(21) Numéro de dépôt: 11703915.6
(22) Date de dépôt: 11.01.2011
(51) Int. Cl.: C12N 5/071, G01N 33/50

(54) **CELLULES ENDOTHELIALES FELINES ORGANOSPECIFIQUES ET UTILISATIONS**
FELINE ORGANSPEZIFISCHEN ENDOTHELZELLEN UND VERWENDUNGEN
ORGAN-SPECIFIC FELINE ENDOTHELIAL CELLS AND USES

(30) Priorité: 11.01.2010 FR 1000094
(43) Date de publication de la demande: 21.11.2012
(73) Titulaire: Centre National de la Recherche Scientifique - CNRS -, 75794 Paris Cedex 16 (FR); Ecole Nationale Veterinaire D'Alfort, 94700 Maisons-Alfort (FR)
(72) Inventeur: KIEDA, Claudine, 45000 Orléans (FR); PAPROCKA, Maria, 50501 Wroclaw (PL); MITTERAND, Michèle, 45160 Ardon (FR); LAMERANT-FAYEL, Nathalie, 45160 Olivet (FR); BOULOUIS, Henri-Jean, 94210 La Varenne Saint Hilaire (FR); HADDAD, Nadia, 94410 Saint-Maurice (FR); MONTEIL, Martine, 94190 Villeneuve-Saint-Georges (FR)
(74) Mandataire: Novagraaf Technologies
(86) Numéro de dépôt international: PCT/FR2011/050037
(87) Numéro de publication internationale: WO 2011/083285

(56) Documents cités:
- FLETCHER N F ET AL: "Growth and characterisation of a cell culture model of the feline blood-brain barrier", VETERINARY IMMUNOLOGY AND IMMUNOPATHOLOGY, AMSTERDAM, NL LNKD- DOI:10.1016/J.VETIMM.2005.08.025, vol. 109, no. 3-4, 15 février 2006 (2006-02-15), pages 233-244, XP024999008, ISSN: 0165-2427 [extrait le 2006-02-15] cité dans la demande
- STEFFAN ANNE-MARIE ET AL: "Productive infection of primary cultures of endothelial cells from the cat liver sinusoid with the feline immunodeficiency virus", HEPATOLOGY, vol. 23, no. 5, 1996, pages 964-970, XP002592502, ISSN: 0270-9139
- PROULX S ET AL: "Transplantation of a tissue-engineered corneal endothelium reconstructed on a devitalized carrier in the feline model", INVESTIGATIVE OPHTHALMOLOGY AND VISUAL SCIENCE 2009 ASSOCIATION FOR RESEARCH IN VISION AND OPHTHALMOLOGY INC. USA LNKD- DOI:10.1167/IOVS.08-2793, vol. 50, no. 6, 2009, pages 2686-2694, XP002592503,
- LUO W -J ET AL: "Cloning, expression and functional analyses of human platelet-derived growth factor-B chain peptide for wound repair of cat corneal endothelial cells", CHINESE JOURNAL OF TRAUMATOLOGY - ENGLISH EDITION 20090201 CN LNKD- DOI:10.3760/CMA.J.ISSN.1008-1275.2009.01.0 06, vol. 12, no. 1, 1 février 2009 (2009-02-01), pages 31-37, XP009136353, ISSN: 1008-1275
- BREITSCHWERDT ET AL: "Feline bartonellosis and cat scratch disease", VETERINARY IMMUNOLOGY AND IMMUNOPATHOLOGY, AMSTERDAM, NL LNKD- DOI:10.1016/J.VETIMM.2008.01.025, vol. 123, no. 1-2, 15 mai 2008 (2008-05-15), pages 167-171, XP022654570, ISSN: 0165-2427 [extrait le 2008-01-19]

## Description

### DESCRIPTION

### Domaine technique

La présente invention se rapporte à des cellules endothéliales félines organospécifiques isolées. La présente invention se rapporte également à des procédés de criblage de molécules, d'étude de pathologies et de production d'agents pathogènes les utilisant.

La présente invention trouve des applications notamment dans le domaine médical et vétérinaire, en particulier dans le domaine thérapeutique, dans le domaine de l'étude des mécanismes cellulaires, dans le domaine de la recherche de nouvelles molécules thérapeutiques et/ou dans le domaine de l'étude des microorganismes.

Dans la description ci-dessous, les références entre crochets **([ ])** renvoient à la liste des références présentées à la fin du texte.

### Etat de la technique

Il est connu dans l'état de la technique que des microorganismes, induisant des pathologies, tels que des bactéries, virus etc. peuvent être transmises d'un mammifère à un autre, incluant l'Homme.

Par exemple, depuis sa découverte en 1992, dans l'article Regnery RL, Anderson BE, Clarridge JE, Rodriguez-Barradas MC, Jones DC, Carr JH: Characterization of a novel Rochalimaea species, R. henselae sp. nov., isolated from blood of a febrile, human immunodefeciency virus-positive patient. J Clin Microbiol 1992, 30:265-274**[1]** *Bartonella henselae* est considérée comme le principal responsable de la lymphoréticulose bénigne d'inoculation chez les humains comme décrit dans Boulouis HJ, Haddad N, Vayssier-Taussat M, Maillard R, Chomel B: Persistent Bartonella infection: epidemiological and clinical implications. Bull Acad Natl Med 2007, 191:1037-10 1044 **[2].** Cette bactérie intracellulaire est responsable de formes plus sérieuses de la maladie chez les patients immunodéprimés, telles que l'angiomatose bacillaire (BA : « bacillary angiomatosis ») et la péliose bacillaire (BP : « bacillary peliose »), caractérisées par la prolifération pseudotumorale des cellules endothéliales des petits vaisseaux sanguins. Ces lésions vasculaires rares se produisent principalement ou exclusivement dans la peau, le foie et la rate comme décrit dans Koehler JE, Sanchez MA, Garrido CS, Whitfeld MJ, Chen FM, Berger TG, Rodriguez- Barradas MC, Leboit PE, Tappero JW: Molecular epidemiology of Bartonella infections in patients with bacillary angiomatosis-peliosis. N Engl J Med 1997, 337:1876-1883**[3]**. Il a été indiqué dans Boulouis HJ, Chang CC, Henn JB, Kasten RW, Chomel BB: Factors associated with the rapid emergence of zoonotic Bartonella infections. Vet Res 2005, 36:383-410 **[4]** que les chats sont le principal réservoir de cette bactérie zoonotique. Les chats immunocompétents ou immunodéprimés, infectés soit naturellement ou soit expérimentalement, ne montrent habituellement aucun signe clinique d'infection comme l'a décrit Yamamoto K, Chomel BB, Kasten RW, Hew CM, Weber DK, Lee WI: Experimental infection of specific pathogen free (SPF) cats with two different strains of Bartonella henselae type I: a comparative study. Vet Res 2002, 3:669-684 **[5]** et Breitschwerdt EB: Feline bartonellosis and cat scratch disease. Vet Immunol Immunopathol 2008, 123:167-71 **[6].**

Deux génotypes (I et II) de *B. henselae* ont été décrits dans Bergmans AM, Schellekens J, van Embden J, Schouls LM: Prédominance 1 of two Bartonella henselae variants among cat-scratch disease patients in the Netherlands. J Clin Microbiol 1996, 34:254-260 **[7]** sur la base de l'analyse des séquences des ARNr 16S. Des études épidémiologiques suggèrent fortement que le génotype I serait plus virulent chez les humains que le génotype II, comme décrit dans Bergmans AM et al.**[7]** ; Sander A, Ruess M, Deichmann K: Two different genotypes of Bartonella henselae in children with cat-scratch disease and their pet cats. Scand J Infect Dis 1998, 30:387-391 **[8];** Sander A, Posselt M, Bohm N, Ruess M, Altwegg M: Detection of Bartonella henselae DNA by two different PCR assays and determination of the genotypes of strains involved in histologically defined cat scratch disease. J Clin Microbiol 1999, 37:993-997 **[9] ;** Fournier PE, Robson J, Zeaiter Z, Mc Dougall R, Byrne S, Raoult D: Improved culture from lymph nodes of patients with cat scratch disease and genotypic characterization of Bartonella henselae isolates in Australia. J Clin Microbiol 2002, 40:3620-3624 [**10]** ;

Dillon B, Valenzuela J, Don R, Blanckenberg D, Wigney DI, Malik R, Morris AJ, Robson JM, Iredell J: Limited diversity among human isolates of Bartonella henselae. J Clin Microbiol 2002, 40:4691- 4699 [**11**] ; Woestyn S, Olivé N, Bigaignon G, Avesani V, Delmée M: Study of genotypes and virB4 secretion gene of Bartonella henselae strains from patients with clinically defined cat scratch disease. J Clin Microbiol 2004, 42:1420-1427 [**12**] et Bouchouicha R, Durand B, Monteil M, Chomel B, Berrich M, Birtles R, Breitschwerdt E, Koehler J, Kasten R, Petit E, Maruyama S, Arvand M, Boulouis H-J, Haddad N: Epidemiological applications of Multi-locus Variable number tandem 1 repeat Analysis (MLVA) for Bartonella henselae of Human and Feline origins. Emerg Infect Dis 2009, 15: 813-816 [**13**]) Jusqu'à présent, aucune étude expérimentale n'a confirmé cette hypothèse.

Il a été démontré histologiquement dans LeBoit PE, Berger TG, Egbert BM, Beckstead JH, Yen TSB, Stoler MH: Bacillary angiomatosis: The histopathology and differential diagnosis of a pseudoneoplastic infection in patients with human immunodeficiency virus disease. Am J Surg Pathol 1989, 13:909-920 **[14]** la présence de micro-colonies de *Bartonella* adjacentes aux cellules endothéliales en prolifération, indiquant que des interactions *Bartonella -* cellules endothéliales (ECs : « Endothelial Cells ») pouvaient être impliquées dans le processus proangiogénique, menant aux lésions vasculaires.

Des approches basées sur la culture de cellules endothéliales primaires dérivées de la macrovascularisation provenant de la veine ombilicale humaine (HUVEC: « Human Umbilical Vein Endothelial Cells ») ont été utilisées pour identifier les facteurs de virulence de *B. henselae.* L'adhésine A (BadA) de *Bartonella,* à l'origine décrite comme "pilus" dans Batterman HJ, Peek JA, Loutit JS, Falkow S, Tompkins LS: Bartonella henselae and Bartonella quintana adherence to and entry into cultured human epithelial cells. Infectlmmun 1995, 63:4553-4556 [**15**] est importante pour la pathogénicité, comme l'indique la publication scientifique Riess T, Raddatz G, Linke D, Schäfer A, Kempf VAJ: Analysis of Bartonella adhesin A expression reveals differences between various B. henselae strains. Infect Immun 2007, 75:35-43 [**16**]. En effet, BadA est impliquée dans l'adhésion aux protéines de la matrice extracellulaire et aux ECs, lors de l'activation du facteur 1 inductible par l'hypoxie (Hypoxy Inducible factor 1/ HIF1) et lors de la sécrétion de cytokines pro-angiogéniques tel que décrit dans Riess T, Andersson SG, Lupas A, Schaller M, Schäfer A, Kyme P, Martin J, Wälzlein JH, Ehehalt U, Lindroos H, Schirle M, Nordheim A, Autenrieth IB, Kempf VA: Bartonella adhesin A mediates a proangiogenic host cell response. J Exp Med 2004, 200:1267-1278 [**17**].

D'autres études ont suggéré que le processus par lequel *B*. *henselae pourrait induire* la prolifération des cellules endothéliales (ECs : « Endothelial Cells) implique très probablement le relargage de facteurs bactériens comme indiqué dans Conley T, Slater L, Hamilton K: Rochalimaea species stimulate human endothelial cell proliferation and migration in vitro. J Lab Clin Med 1994, 124:521-528 [**18**] **;** Palmari J, Teysseire N, Dussert C, Raoult D: Image cytology and topographical 1 analysis of proliferation of endothelial cells in vitro during Bartonella (Rochalimaea) infection. Analytical Cell Pathol 1996, 11:13-30 [**19**] **;** Maeno NH, Oda K, Yoshiie MR, Wahid TF, Matayoshi S: Live Bartonella henselae enhances endothelial cell proliferation without direct contact. Microb Pathog 1999,7 27:419-427 **[20]** ; McCord AM, Cuevas J, Anderson BE: Bartonella-induced endothelial cell proliferation is mediated by release of calcium from intracellular stores. DNA Cell Biol 2007, 26:657-11 663 **[21].**

Il a également été montré que des facteurs de l'hôte jouent un rôle *in vitro* dans les interactions de *B. henselae*-host conduisant à l'angiogenèse. D'après McCord AM, Burgess AWO, Whaley MJ, Anderson BE: Interaction of Bartonella henselae with endothelial cells promotes monocyte/macrophage chemoattractant protein 1 gene expression and protein production and triggers monocyte migration. Infect Immun 2005, 73:5735-5742 [**22**] les cellules endothéliales infectées peuvent induire l'expression et la production de protéines pro-angiogéniques. Les études de l'expression du Facteur de Croissance de l'Endothéliu Vasculaire (VEGF pour « Vascular Endothelial Growth Factor ») dans des échantillons cliniques décrits dans Kempf VA, Volkmann B, Schaller M: Evidence of a leading role for VEGF in Bartonella henselae-induced endothelial cell proliferations. Cell Microbiol 2001, 3:623-632 [**23**] ou dans des cultures de cellules endothéliales (ECs pour Endothelial Cells) comme décrites dans Maneo et al. [**20**], Kempf et al. [**23**] et Resto-Ruiz SI, Schmiederer M, Sweger D, Newton C, Klein TW, Friedman H, Anderson BE: Induction of a potential paracrine angiogenic loop between human THP-1 macrophages and human microvascular endothelial cells during Bartonella henselae infection. Infect Immun 2002, 70:4564-4570 [**24**] suggèrent en effet la production exogène de VEGF, et l'implication de l'augmentation de l'expression des récepteurs au VEGF, en particulier le VEGFR-2, par les ECs comme décrit dans Ferrara N, Gerber HP, Le Couter J: The biology of VEGF and its receptors. Nature Medicine 2003, 9:669-676 [**25**]. De plus, l'activité anti-apoptotique de la molécule BepA de *B. henselae,* molécule associée au système de sécrétion de type IV , dans les cellules endothéliales de la veine ombilicale humaine est corrélée à une augmentation importante du niveau de l'adénosine monophosphate 3', 5'-cyclique (AMPc).Schmid MC, Scheidegger F, Dehio M, Balmelle-Devaux N, Schulein R, Guye P, Chennakesava CS, Biedermann B, Dehio C: A translocated bacterial protein protects vascular endothelial cells from apoptosis. PLoS Pathog 2006, 2:1083-1096 **[26]**

Cependant, ces études sont exclusivement basées sur l'utilisation des HUVEC dérivées de la macrovascularisation. Ces cellules sont très différentes des EC dérivées de la microvascularisation impliquées dans les pathologies précitées.

Le document Steffan et al. « Productive infection of primary cultures of endothelial cells from the cat liver sinusoid with the feline immunodeficiency virus. "Hepatology. 1996 May;23(5):964-70. décrit un procédé et l'isolement de cellules endothéliales sinusoïdale de foie de félins.Ce document décrit également que les cellules isolées expriment le facteur de Von Willebrand, un récepteur « scavenger » et sont capables d'incorporer des lipoprotéines acétylés de faible densité.

Il existe donc un réel besoin d'isoler, de caractériser et de pouvoir utiliser des cellules endothéliales isolées de la microvascularisation.

Il existe donc un réel besoin de trouver des cellules endothéliales impliquées dans ces processus pour pouvoir étudier les mécanismes cellulaires, par exemple les mécanismes d'interactions entre *B. henselae* et l'endothélium vasculaire.

De plus les cellules utilisées dans l'art antérieur sont des cellules humaines qui ne permettent pas de savoir pourquoi des microorganismes non pathogènes pour des félins par exemple, le sont pour l'être humain. Il n'existe pas actuellement de lignées cellulaires établies de cellules endothéliales félines.

Il existe donc un réel besoin d'isoler, de caractériser et de pouvoir utiliser des cellules endothéliales isolées afin d'étudier les mécanismes cellulaires et moléculaires impliqués dans la colonisation de ces cellules par des microorganismes et/ou d'identifier les éléments de la cellule qui favorisent et/ou inhibent le développement de ces microorganismes.

Il existe également un réel besoin d'isoler, de caractériser et de pouvoir utiliser des cellules endothéliales isolées afin d'étudier les mécanismes cellulaires et moléculaires impliqués dans la colonisation de ces cellules par des microorganismes.

En outre, il n'existe pas dans l'état de la technique d'études comparatives de l'interaction entre des microorganismes, par exemple *B.* henselae et des cellules endothéliales humaines ou des cellules endothéliales félines, sachant que des microorganismes, comme *B*. *henselae,* peuvent être zoonotiques.

Il existe donc un réel besoin d'isoler et d'identifier des cellules endothéliales félines afin de pouvoir comparer les mécanismes d'interactions impliqués entre les cellules endothéliales félines et des microorganismes et entre les cellules endothéliales humaines et des microorganismes.

Par ailleurs, il existe dans l'état de la technique, un réel besoin d'isoler et d'identifier des cellules endothéliales pour cribler des molécules afin d'identifier celles qui sont susceptibles d'inhiber, de stopper et/ou de détruire les microorganismes pathogènes spécifiques ou non des cellules endothéliales.

Enfin, il existe un réel besoin dans l'état de la technique d'isoler et d'identifier des cellules endothéliales pour cultiver des microorganismes, par exemple des microorganismes zoonotiques.

Par ailleurs, il est également connu dans l'état de la technique que des microorganismes sont spécifiques de certaines cellules et de certain mammifère. Par exemple, l'hémoparasite épicellulaire *Haemobartonella felis,* actuellement *Mycoplasma harmofelis* et *Mycoplasma haemominutum,* qui provoque la maladie nommée anémie infectieuse féline est reconnu comme étant une des causes les plus fréquentes des anémies hémolytiques chez les chats domestiques. Cependant, il reste dans l'état de la technique de nombreuses questions sans réponse concernant son mode de transmission, sa prévalence et son impact clinique sur les populations de félins domestiques. Un autre problème de l'état de la technique est de trouver des moyens efficaces d'identification de ces microorganismes.

Il existe donc un réel besoin d'isoler et d'identifier des cellules endothéliales félines afin de pouvoir étudier les microorganismes spécifiques de ces cellules et/ou d'identifier des molécules thérapeutiques permettant le traitement des pathologies associées à ces microorganismes et/ou d'inhiber et /ou détruire ces microorganismes.

### Exposé de l'invention

La présente invention permet précisément de résoudre les problèmes et inconvénients précités de l'état de la technique en fournissant des cellules endothéliales félines organospécifiques.

La présente invention concerne également des cellules endothéliales félines de la macrovascularisation et de la microvacularisation et leur caractérisation en tant que cellules endothéliales.

Les inventeurs sont les tous premiers à avoir réussi à isoler et établir des lignées cellulaires de cellules endothéliales félines organospécifiques.

En outre, les inventeurs ont immortalisé lesdites cellules et ont obtenu des lignées cellulaires établies, c'est à dire des lignées cellulaires immortalisées, stables, non tumorigènes et dont les caractéristiques sont identiques d'une génération à une autre. Une définition d'une lignée cellulaire établie est donnée dans le livre Gene IV, de Benjamin Leuvine, page 1133, 6ème édition, De Boeck Université.

La présente invention a donc pour objet une cellule endothéliale féline organospécifique isolée comprenant le facteur de Von Willebrand, l'enzyme de conversion de l'angiotensine et les clusters de différentiation (CD) CD 31, 34, 105, 54, 62E, 62P, 146 choisie parmi les cellules déposées à la Collection Nationale de Culture de Microorganismes (CNCM), sous les numéros CNCM n°I-4250 (F Int MEC), n° I-4251 (F PLN MEC), n° I-4252 (FOm EC), n° I-4253 (F Li MEC)n° I-4254 (F Sk MEC),n° I-4255 (F PP MEC), n° I-4256 (F He MEC), n° I-4257 (F Br MEC), n° I-4258 (F Lu MEC).

La présente invention a également pour objet un ensemble de cellules endothéliales félines organospécifiques isolées comprenant le facteur de Von Willebrand, l'enzyme de conversion de l'angiotensine et les clusters de différentiation (CD) CD 31, 34, 105, 54, 62E, 62P, 146.

Les cellules endothéliales félines organospécifiques isolées selon la présente invention ont été déposées à la Collection Nationale de Culture de Microorganismes (CNCM), Institut Pasteur, 25 rue du Docteur Roux, 75724 PARIS Cedex 15, le 24 novembre 2009, sous les numéros CNCM n°I-4250 (F Int MEC), n° I-4251 (F PLN MEC), n° I-4252 (F Om EC), n° I-4253 (F Li MEC)n° I-4254 (F Sk MEC),n° I-4255 (F PP MEC), n° I-4256 (F He MEC), n° I-4257 (F Br MEC), n° I-4258 (F Lu MEC).

Dans la présente par « organospécifique » on entend des cellules représentant la microvascularisation ou la macrovascularisation de chacun des organes dont elles sont issues.

Les inventeurs ont mis en évidence que selon l'origine tissulaire, les cellules endothéliales diffèrent et sont la clé de la spécificité du ciblage d'un organe ou tissu en contrôlant les mécanismes moléculaires et cellulaires qui régissent, par exemple l'entrée dans le tissu sous-jacent à la paroi endothéliale. Les inventeurs ont également mis en évidence que les cellules endothéliales de la microvascularisation sont distinctes et ont donc un comportement moléculaire et biologique différent des cellules endothéliales de la macrovascularisation.

La présente invention a également pour objet des cellules endothéliales félines isolées organospécifiques dérivées de la microvascularisation choisies parmi les cellules endothéliales félines isolées organospécifiques dérivées de la microvascularisation déposées à la Collection Nationale de Culture de Microorganismes (CNCM), Institut Pasteur, 25 rue du Docteur Roux, 75724 PARIS Cedex 15, le 24 novembre 2009 sous les numéros CNCM n°I-4250 (F Int MEC), n° I-4251 (F PLN MEC), n° I-4252 (F Om EC), n° I-4253 (F Li MEC)n° I-4254 (F Sk MEC),n° I-4255 (F PP MEC ), n° I-4256 (F He MEC), n° I-4257 (F Br MEC), n° I-4258 (F Lu MEC).

La présente invention a également pour objet des cellules endothéliales félines isolées organospécifiques dérivées de la macrovascularisation choisies parmi cellules endothéliales félines isolées organospécifiques dérivées de la macrovascularisation déposées à la Collection Nationale de Culture de Microorganismes (CNCM), Institut Pasteur, 25 rue du Docteur Roux, 75724 PARIS Cedex 15, le 24 novembre 2009 sous les numéros CNCM n°I-4250 (F Int MEC), n° I-4251 (F PLN MEC), n° I-4252 (F Om EC), n° I-4253 (F Li MEC)n° I-4254 (F Sk MEC),n° I-4255 (F PP MEC), n° I-4256 (F He MEC), n° I-4257 (F Br MEC), n° I-4258 (F Lu MEC).

La présente invention a également pour objet les cultures cellulaires comprenant les cellules de la présente invention.

Les inventeurs ont également mis en évidence que l'incubation des cellules de l'invention avec des composés particuliers, par exemple des facteurs de stimulation de l'angiogenèse, permettait la prolifération et le développement d'un tissu vasculaire.

L'invention a donc également pour objet un procédé de criblage de molécules susceptibles d'induire une différentiation et/ou une spécialisation d'une cellule endothéliale féline comprenant les étapes suivantes :
- introduction d'au moins une molécule à cribler dans un milieu adapté à la culture de ladite cellule,
- introduction d'au moins une cellule endothéliale féline isolée selon l'invention dans ledit milieu,
- culture desdites cellules dans ledit milieu pendant un temps suffisant pour permettre la différenciation et/ou la spécialisation desdites cellules,
- prélèvement d'au moins une cellule cultivée dudit milieu de culture,
- observation de la différentiation et/ou spécialisation de ladite cellule par observation phénotypique de la cellule et/ou par révélation de marqueurs de différentiation.

Dans le procédé de criblage de molécules, la cellule endothéliale féline est une cellule endothéliale féline organospécifique isolée comprenant le facteur de Von Willebrand, l'enzyme de conversion de l'angiotensine et les clusters de différentiation (CD) CD 31, 34, 105, 54, 62E, 62P, 146. choisie dans le groupe comprenant les cellules déposées à la Collection Nationale de Culture de Microorganismes (CNCM), Institut Pasteur, 25 rue du Docteur Roux, 75724 PARIS Cedex 15, le 24 novembre 2009 sous les numéros CNCM n°I-4250 (F Int MEC), n° I-4251 (F PLN MEC), n° I-4252 (F Om EC), n° I-4253 (F Li MEC)n° I-4254 (F Sk MEC),n° I-4255 (F PP MEC), n° I-4256 (F He MEC), n° I-4257 (F Br MEC), n° I-4258 (F Lu MEC).

La « molécule à cribler », peut être par exemple une molécule chimique, un peptide, une hormone, une molécule d'acide nucléique, un extrait végétal. Par exemple, la molécule à cribler peut être une molécule disponible dans le commerce et/ou pour laquelle on désire connaître son effet sur les cellules de la présente invention, par exemple le facteur de croissance vasculaire (VEGF : « Vascular Endothelial Growth Factor »), le facteur de perméabilité vasculaire (VPF : « Vascular Permeability Factor »), les membres de la famille du facteur de croissance épidermique (EGF : « Epidermal Growth Factor »).

Il peut s'agir également de molécules connues de l'homme du métier qui sont susceptibles d'induire, ou d'inhiber une angiogénèse. Par exemple, il peut s'agir des molécules choisies dans le groupe comprenant le VEGF, les angiopoïétines-1 et 2.

Selon la présente invention, le « milieu de culture approprié » peut être tout milieu de culture connu de l'homme du métier adapté à la culture de cellule endothéliale féline. Il peut s'agir par exemple d'un milieu de culture disponible dans le commerce. Il peut s'agir par exemple d'un milieu de culture décrit dans Bizouarne N, Denis V, Legrand A, Monsigny M, Kieda C: A SV-40 immortalized murine endothelial cell line from peripheral lymph node high endothelium expresses a new alpha-I-fucose binding protein. Biol Cell 1993, 79:209-218 [27], de milieux commercialisés par la société Promocell, le milieu DMEM (marque déposée), DMEM/F-12 (marque déposée), F-10 (marque déposée), F-12 (marque déposée), MEM (marque déposée), RPMI (marque déposée), OptiMEM (marque de commerce) commercialisés par la société Invitrogen.

Selon l'invention, le milieu de culture peut être supplémenté en outre avec un fongicide, par exemple la fongizone (marque de commerce) ; un antibiotique, par exemple la gentamicine (marque de commerce) ; de sérum de veau foetal. L'homme du métier grâce à ses connaissances générales saura aisément adapter le milieu de culture.

Selon l'invention, les cellules endothéliales félines peuvent être cultivées à une température comprise par exemple entre 5°C et 45°C, de préférence à une température comprise entre 17 et 40 °C, de préférence à une température de 37°C .

Selon l'invention, les cellules endothéliales félines peuvent être cultivées dans une atmosphère comprenant un pourcentage de CO₂ compris entre 2 et 10%, de préférence entre 4 et 6%, de manière encore plus préférée de 5%.

Selon l'invention, les cellules endothéliales félines peuvent être cultivées dans une atmosphère humide, par exemple une atmosphère avec un taux d'humidité compris entre 50 et 90%, de préférence entre 60 et 85%.

Selon l'invention, la révélation de marqueurs de différentiation peut être effectuée par tout moyen et/ou procédé connu de l'homme du métier. Par exemple, il peut s'agir de l'utilisation d'anticorps spécifiques desdits marqueurs ou d'autres moyens connus de l'homme du métier. Par exemple, il peut s'agir d'anticorps disponibles dans le commerce, d'anticorps capables de faire une réaction croisée avec le chat, par exemple il peut s'agir des anticorps décrits dans le travail de Fletcher N.F. et al (2006) **[31]** et Kieda C. et al, (2002) **[29]** par exemple des anticorps disponibles dans le commerce, par exemple des anticorps monoclonaux, par exemple des anticorps monoclonaux de souris (anti CD49e ou anti CD29) commercialisés par la société Serotec, la société Sigma, la société Becton Dickinson Biosciences, des anticorps polyclonaux (anticorps de lapin anti Facteur de Von Willebrand Dako, Cambridge UK-).

Dans la présente, la culture de cellules peut être réalisée dans tout contenant connu de l'homme du métier, par exemple, il peut s'agir de boîtes de Pétri, de plaques 6 puits, 24 puits, 96 puits, de tubes à essai.

Dans la présente l'étape de prélèvement peut être réalisée par toute méthode connue de l'homme du métier. Par exemple, le prélèvement peut être réalisé par un dispositif de prélèvement, par exemple un robot ClonePix ou ClonePixFL commercialisés par la société PROTEIGENE, un tube de prélèvement destiné à l'analyse par cytofluorimétrie.

Dans la présente l'observation phénotypique peut être réalisée par toute méthode connue de l'homme du métier. Par exemple, elle peut être réalisée par observation directe, par observation via un microscope, par exemple un microscope optique, un microscope électronique à balayage. Il peut s'agir par exemple d'un vidéo microscope Zeiss axiovert 200M (marque déposée), un microscope Nikon TMS (marque déposée).

La présente invention a également pour objet un kit de mise en oeuvre du procédé de la présente invention comprenant au moins une cellule endothéliale féline isolée de la présente invention et un moyen de détection de la différentiation et/ou spécialisation cellulaire.

Avantageusement, les cellules endothéliales de l'invention peuvent être utilisées pour étudier les mécanismes cellulaires et/ou moléculaires de l'infection d'une cellule par un microorganisme.

Ainsi, la présente invention a également pour objet un procédé d'étude *in vitro* de pathologies dues à un microorganisme pathogène comprenant les étapes suivantes :
- culture d'au moins une cellule endothéliale féline selon l'invention dans un milieu de culture approprié,
- inoculation de ladite cellule avec un microorganisme pathogène,
- culture des cellules inoculées, et
- observation d'un ou de plusieurs des critères suivants : évolution du phénotype des cellules, évolution de la cytotoxicité, induction de l'apoptose, stimulation de la croissance angiogenèse.

Dans la présente invention, on entend par « microorganisme » les bactéries, les virus, les champignons, les levures et les protozoaires.

Dans le procédé d'étude in vitro de pathologies, le microorganisme pathogène peut être un microorganisme pathogène spécifique des cellules endothéliales félines ou non. Par exemple, il peut s'agir de microorganismes pathogènes pour des cellules humaines, de microorganismes pathogènes pour les cellules humaines et félines et de microorganismes pathogènes uniquement pour les cellules félines. Il peut s'agir d'un microorganisme spécifique des cellules endothéliales félines microvasculaires et/ou un microorganisme spécifique des cellules endothéliales félines macrovasculaires ou non, et/ou d'un microorganisme non spécifique de cellules endothéliales félines.

Il peut s'agir par exemple d'un microorganisme spécifique des cellules endothéliales félines choisi dans le groupe comprenant *Bartonella henselae, Mycoplasma haemofelis, Mycoplasma haemominutum, Anaplasma phagocyfophilum et Rickettsia felis.*

Dans le procédé d'étude *in vitro* de pathologies, la cellule endothéliale féline est une cellule endothéliale féline organospécifique isolée comprenant le facteur de Von Willebrand, l'enzyme de conversion de l'angiotensine et les clusters de différentiation (CD) CD 31, 34, 105, 54, 62E, 62P, 146 choisie dans le groupe comprenant les cellules déposées à la Collection Nationale de Culture de Microorganismes (CNCM), Institut Pasteur, 25 rue du Docteur Roux, 75724 PARIS Cedex 15, le 24 novembre 2009 sous les numéros CNCM n°I-4250 (F Int MEC), n° I-4251 (F PLN MEC), n° I-4252 (F Om EC), n° I-4253 (F Li MEC)n° I-4254 (F Sk MEC),n° I-4255 (F PP MEC), n° I-4256 (F He MEC), n° I-4257 (F Br MEC), n° I-4258 (F Lu MEC)..

Dans la présente invention, par cytotoxicité il est entendu une altération des cellules, par exemple une altération génomique, physique et/ou toute altération cellulaire connue de l'homme du métier.

Dans la présente invention, par apoptose il est entendu la mort programmée de la cellule, c'est-à-dire le déclenchement de mécanismes cellulaires conduisant à la mort de la cellule.

Dans la présente invention, l'étape d'observation peut être effectuée par toute méthode et/ou moyen connus de l'homme du métier, par exemple, par les méthodes décrites précédemment. L'homme du métier de par ses connaissances générales saura aisément choisir et adapter, le cas échéant, les méthodes et/ou moyens connus dans l'état de la technique pour réaliser cette étape. Par exemple, l'observation de l'induction de l'apoptose peut être réalisée par visualisation au microscope de la mort cellulaire, par détection dans le milieu de culture de molécules impliquées dans les procédés apoptotiques, par exemple à l'aide d'anticorps spécifiques, ou par l'observation de la condensation nucléaire par coloration Hoescht/DAPI. L'observation de la stimulation de la croissance angiogénique peut être réalisée, par exemple, par visualisation au microscope de structures vasculaires, par détection de molécules et/ou de marqueurs spécifiques de l'angiogenèse par exemple avec des anticorps spécifiques.

Avantageusement, le procédé d'étude *in vitro* de pathologies permet également de déterminer la pathogénicité du microorganisme vis-à-vis de la cellule endothéliale. Si le microorganisme est pathogène, la croissance de la cellule endothéliale est altérée par exemple une mort cellulaire ou augmentation de sa durée de vie), si le microorganisme n'est pas pathogène, il est sans effet sur la cellule.

Avantageusement, le procédé de l'invention permet également d'étudier la spécificité et le ciblage d'un agent pathogène vis-à-vis d'une catégorie de cellule endothéliale, à savoir une cellule endothéliale de la micro ou macrovascularisation. Ainsi la présente invention permet donc avantageusement d'identifier les microorganismes pathogènes spécifiques de cellules endothéliales microvasculaires ou macrovasculaires.

Les inventeurs ont également démontré que les cellules de la présente invention peuvent être utilisées dans des procédés de criblage de molécules susceptibles d'avoir une action sur un microorganisme pathogène vis à vis d'une cellule endothéliale féline.

L'invention a donc également pour objet un procédé de criblage de molécules susceptibles d'avoir une action sur un microorganisme pathogène vis-à-vis d'une cellule endothéliale féline comprenant les étapes suivantes :
- introduction d'au moins une molécule à cribler dans un milieu adapté à la culture de ladite cellule,
- introduction d'au moins une cellule endothéliale féline dans ledit milieu,
- introduction du microorganisme pathogène dans ledit milieu,
- culture des cellules inoculées, et
- observation du développement ou non du microorganisme.

Dans le procédé de l'invention, la cellule endothéliale féline est une cellule endothéliale féline organospécifique isolée comprenant le facteur de Von Willebrand, l'enzyme de conversion de l'angiotensine et les clusters de différentiation (CD) CD 31, 34, 105, 54, 62E, 62P, 146 choisie dans le groupe comprenant les cellules déposées à la Collection Nationale de Culture de Microorganismes (CNCM), Institut Pasteur, 25 rue du Docteur Roux, 75724 PARIS Cedex 15, le 24 novembre 2009 sous les numéros CNCM n°I-4250 (F Int MEC), n° I-4251 (F PLN MEC), n° I-4252 (F Om EC), n° I-4253 (F Li MEC)n° I-4254 (F Sk MEC),n° I-4255 (F PP MEC), n° I-4256 (F He MEC), n° I-4257 (F Br MEC), n° I-4258 (F Lu MEC)..

Dans le procédé de criblage de molécules le microorganisme peut être un microorganisme tel que défini ci-dessus.

Avantageusement, le procédé de l'invention permet d'identifier les molécules actives sur le microorganisme avant infection et/ou les molécules actives sur le microorganisme ayant un effet sur le mécanisme d'infection de la cellule par le microorganisme.

Dans un autre mode de réalisation du procédé de l'invention, l'étape d'introduction de la molécule à cribler peut être réalisée postérieurement à l'étape d'inoculation. Ainsi le procédé de l'invention permet avantageusement d'identifier les molécules susceptibles d'être actives sur le microorganisme, dans la cellule ou à sa surface, après infection.

La présente invention a également pour objet un kit pour la mise en oeuvre du procédé de production de microorganismes pathogènes comprenant au moins une cellule endothéliale féline isolée de l'invention et un moyen d'inoculation de ladite cellule par ledit microorganisme.

En outre, les inventeurs ont également mis en évidence que les cellules endothéliales provenant d'espèces différentes, félin ou humain ont un comportement différent vis-à-vis de pathogènes. Les inventeurs sont les tous premiers à avoir développés des lignées de cellules endothéliales de félins et démontrés les effets *in vitro* de l'infection par des souches de *Bartonella* (*B*. *henselae* genotype I / II et *B. tribocorum*) distinctes chez l'homme *versus* chez le félin.

Les inventeurs ont également démontré que l'utilisation des cellules de l'invention dans un procédé d'étude de microorganismes permet avantageusement d'identifier des microorganismes pathogènes spécifiques des cellules endothéliales félines. Les inventeurs ont démontré en outre que des microorganismes ont des effets biologiques différents pour des cellules endothéliales félines de ceux observés vis à vis des cellules endothéliales humaines.

Ainsi les cellules de la présente invention peuvent être utilisées dans des études comparatives d'infection entre des cellules endothéliales autres que les cellules félines, par exemple des cellules endothéliales humaines, et des cellules de l'invention par un microorganisme afin de mettre en évidence, par exemple les différences de pathogénicité du microorganisme d'une cellule à l'autre.

Avantageusement, le procédé d'étude de microorganisme selon l'invention permet également d'étudier la spécificité et le ciblage d'un type endothélial par rapport à l'autre en termes de caractéristiques vasculaires, c'est-à-dire en fonction que les cellules endothéliales sont des cellules de la micro ou de la macrovascularisation.

Les inventeurs ont également mis en évidence que les cellules endothéliales félines de l'invention peuvent être utilisées comme réservoir et/ou comme un moyen permettant de produire des microorganismes, par exemple des microorganismes dépendants des cellules endothéliales félines.

La présente invention a donc également pour objet un procédé in vitro de production de microorganismes pathogènes comprenant les étapes suivantes :
- culture d'au moins une cellule endothéliale féline de l'invention dans un milieu de culture approprié,
- inoculation de ladite cellule cultivée avec ledit microorganisme pathogène,
- la culture des cellules inoculées permettant la prolifération dudit microorganisme pathogène, et
- récupération des microorganismes pathogènes produits.

Dans la présente invention, l'étape de récupération des microorganismes produits peut être effectuée par toute technique et/ou moyen connu de l'homme du métier.

Par ailleurs, les cellules de la présente invention peuvent être utilisées dans tous les procédés connus dans l'état de la technique utilisant des cultures cellulaires et/ou des cellules.

Les cellules de la présente invention trouvent des applications dans les domaines vétérinaire et médical, comme par exemple pour identifier de nouvelles molécules thérapeutiques, étudier des microorganismes pathogènes, identifier des microorganismes pathogènes spécifiques.

Par exemple les cellules de la présente invention peuvent être utilisées afin d'étudier, par exemple l'expression différentielle de facteurs significatifs de pathologies. En outre, les cellules de l'invention peuvent permettre d'étudier des effets en fonction, par exemple de l'origine tissulaire, c'est-à-dire, par exemple si les cellules endothéliales sont des cellules de la micro ou de la macrovascularisation, et/ou, par exemple de comparer les résultats obtenus avec les cellules endothéliales félines de ceux observés vis à vis d'autres espèces, par exemple des cellules humaines.

En outre, les cellules de l'invention peuvent être utilisées, par exemple dans des tests d'inflammations et/ou des tests évaluant les effets possibles de composés d'anti-inflammatoires en fonction en fonction que les cellules endothéliales sont des cellules de la micro ou de la macrovascularisation et/ou, par exemple, de comparer les résultats obtenus avec les cellules endothéliales félines de ceux observés vis à vis des cellules endothéliales d'autres espèces, par exemple des cellules humaines.

D'autres avantages pourront encore apparaître à l'homme du métier à la lecture des exemples ci-dessous, illustrés par les figures annexées, donnés à titre illustratif.

### Brève description des figures

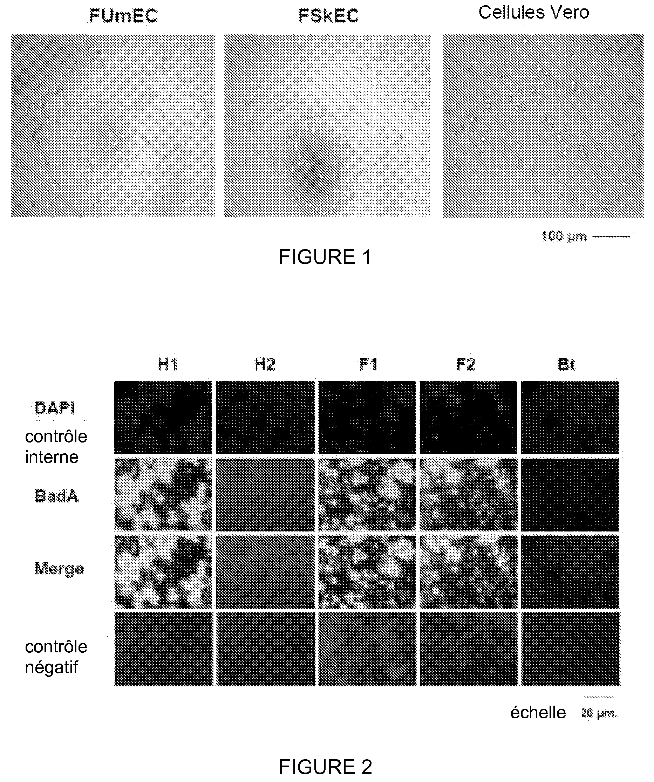

- La figure 1 représente des photographies de l'angiogenèse de cellules endothéliales félines de l'invention 10 heures après mise en culture. FUcEC correspond à la lignée cellulaire de cellules endothéliales félines de la macrovascularisation, FSkEC correspond à la lignée de cellules endothéliales félines de la microvascularisation, Vero cells correspond aux cellules contrôles.
- La figure 2 représente des photographies relevant l'expression de différentes souches de *B. henselae* et de *B. tribocorum.* Différents marqueurs ont été utilisés le diamino-2-phénylindole chlorure (DAPI) : ligne DAPI, un anticorps anti BadA marqué avec un fluorochrome vert : ligne BadA, et les deux colorants mélangés : ligne mélange, et un contrôle négatif pour lequel aucun marqueur n'est utilisé. Des photographies représentant un contrôle négatif ont également été faites. Les différentes souches étaient les souches H1 :souche *B henselae* humain génotype I, H2 : souche *B henselae* humain génotype II, F1 : souche *B henselae* américain féline type I, F2 : souche *B henselae* américain féline type Il et Bt : *B. tribocorum* utilisé comme contrôle.

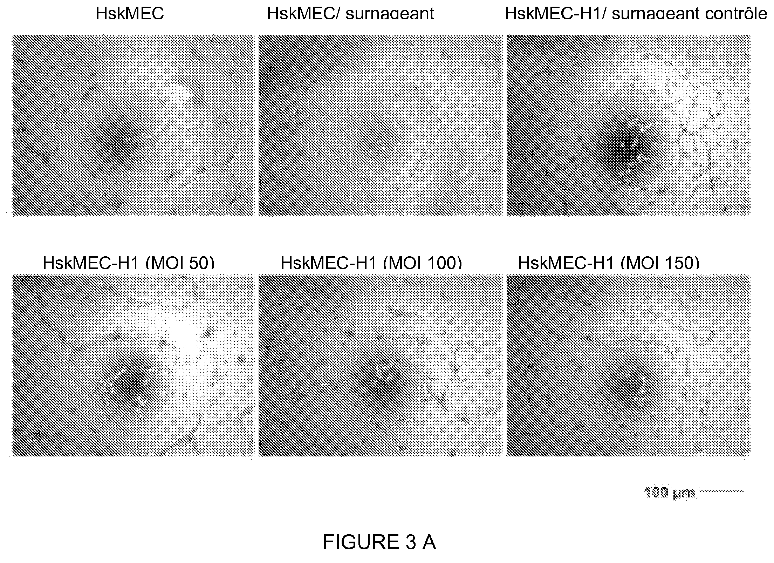

- La figure 3A représente des photographies de culture de cellules endothéliales de l'invention et l'effet de l'infection par la souche *B henselae* humain type I sur la formation de structures capillaires. Cette figure représente l'effet en fonction de la multiplicité d'infection (MOI : « Multiplicity of Infection ») égale à 50, 100 ou 150 MOI.

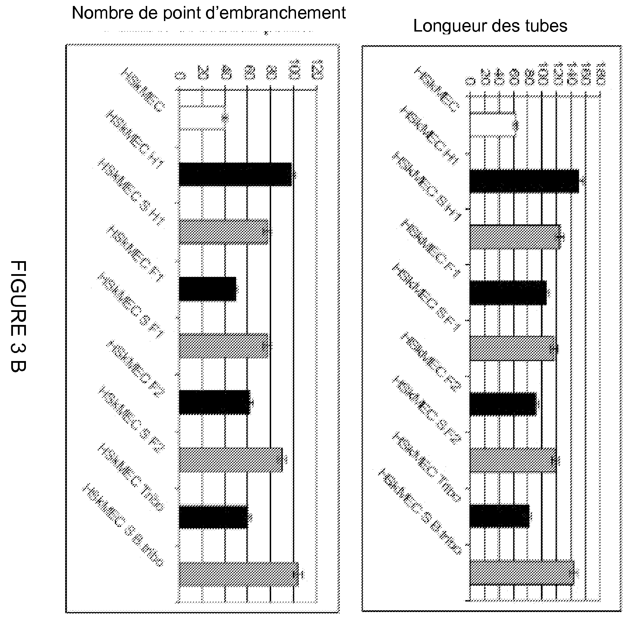

- La figure 3B représente des histogrammes représentant la longueur des tubes en micromètres en fonction des cellules endothéliales humaines microvasculaires de la peau humaine (HSkMEC : « human skin microvascular endothelial cells ») et de leur traitement par les différentes souches bactériennes ou leur surnageant de culture. Soient : peau (HSkMEC : « human skin microvascular endothelial cells ») « human skin microvascular endothelial cells »), à savoir HSkMEC : cellule non infectée, HSkMEC H1 : cellule infectée avec *B*. *henselae* Houston-1 (« strain Houston-1 » (génotype I (H1) / ATCC 49882)), HSkMEC SH1 cellule traitée par le surnageant de culture des H1, HSkMEC F1 cellule infectée par une souche *B. henselae* isolée à partir de chats de génotype I (F1/ Strain 297172), HSkMEC SF1 cellule traitée par le surnageant de culture des F1 , HSkMEC F2 cellule infectée par une souche *B. henselae* isolée à partir de chats de génotype II, HSkMEC SF2 cellule traitée par le surnageant de culture des F2, HSkMEC tribo cellule infectée par *B. tribocorum* (Bt), HSkMEC S tribo cellule traitée par le surnageant de culture des Bt ; et le nombre d'embranchements en fonction de ces mêmes cellules (figure 3b).

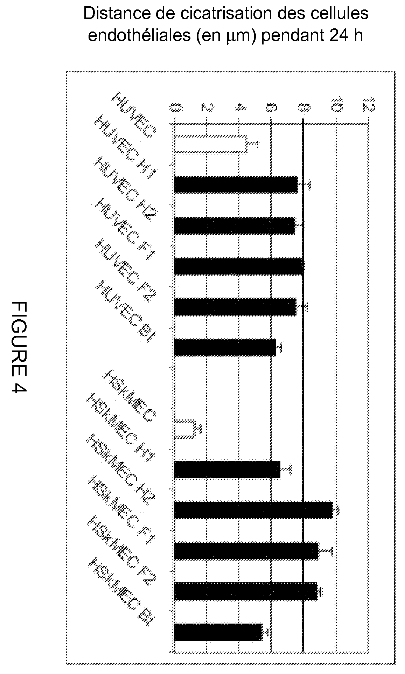

- La figure 4 représente un histogramme indiquant la longueur de cicatrisation en micromètres en 24 heures, de cellules endothéliales humaines isolée de la macrovascularisation de la veine de cordon ombilical (HUVEC : « human umbilical vein endothelial cell ») et une isolée de la microvascularisation, les cellules endothéliales humaines microvasculaires de la peau humaine (HSkMEC : « human skin microvascular endothelial cells ») infectées par différentes souches bactériennes à savoir HSkMEC H1 : cellule HSkMEC infectée avec *B. henselae* Houston-1 (« strain Houston-1 » (génotype I (H1)/ATCC 49882)), HSkMEC F1 : cellule HSkMEC infectée par une souche *B*. *henselae* isolée à partir de chats de génotype I (F1/ Strain 297172), HSkMEC F2 : cellule HSkMEC infectée par une souche *B. henselae* isolée du chat, de génotype II, HSkMEC Bt : cellule HSkMEC infectée par *B. tribocorum* (Bt), HUVEC H1 : cellule HUVEC infectée avec *B. henselae* Houston-1 (« strain Houston-1 » (génotype I (H1) / ATCC 49882)), HUVEC F1 : cellule HUVEC infectée par une souche *B. henselae* isolée du chat, de génotype I (F1/ Strain 297172), HUVEC F2 : cellule HUVEC infectée par une souche *B. henselae* isolée du chat, de génotype II, HUVEC Bt : cellule HSkMEC infectée par *B. tribocorum* (Bt).

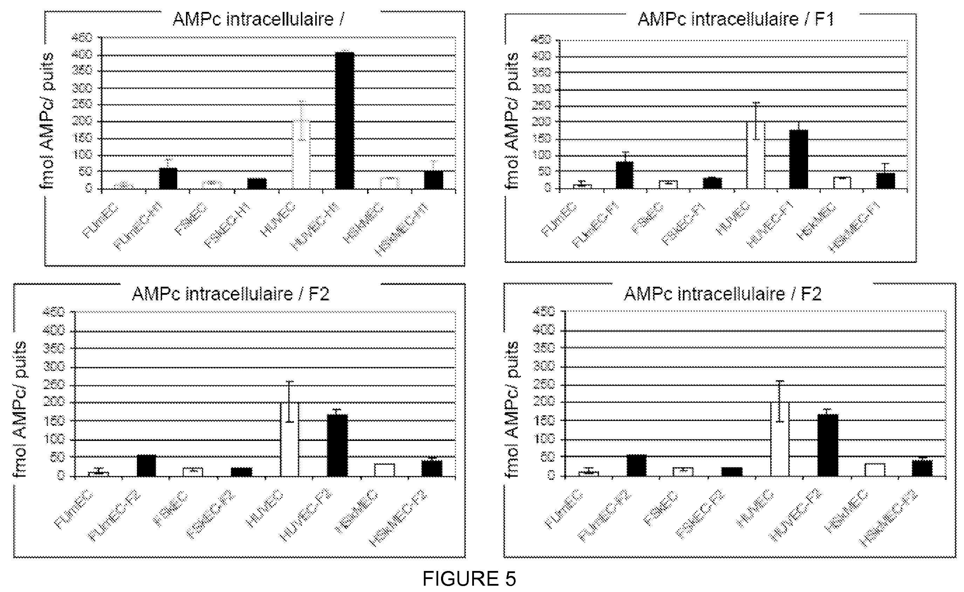

- La figure 5 représente des histogrammes indiquant la production intracellulaire d'AMPc dans les cellules endothéliales humaines de la veine ombilicale (HUVEC : « Human umbilical vein endothelial cell »), les cellules endothéliales humaines de la peau humaine (HSkMEC : « human skin endothelial cells »), cellules endothéliales félines de la peau (FSkMEC) et les cellules endothéliales félines du cordon ombilical (FUcEC) en fonction de leur infection par une souche *B*. *henselae* isolée à partir de chats de génotype I (F1), *B. henselae* de génotype I (H-1) (« strain Houston-1 » génotype I (H1) / ATCC 49882), une souche *B. henselae* de génotype II isolée à partir de chats (FII) et *B. tribocorum* (Bt).

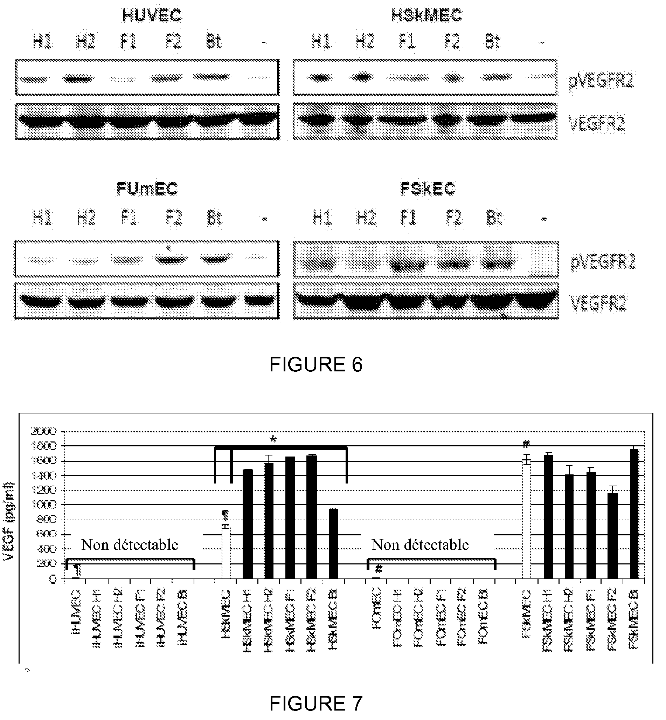

- La figure 6 représente des photographies de buvardage de western (Western Blot) correspondant à l'expression du récepteur 2 du VEGF (VEGFR-2) et du récepteur 2 du VEGF phosphorylé (pVEGFR-2) dans les cellules FSkMEC, FUcEC, HSkMEC, HUVEC infectées par une souche *B. henselae* de génotype I (F1) isolée à partir de chats, *B. henselae* de génotype I (H-1) (« strain Houston-1 » génotype I (H1) / ATCC 49882), *B. henselae* de génotype II (H2), une souche *B*. *henselae* de génotype II isolée à partir de chats (FII) et *B. tribocorum* (Bt).
- La figure 7 représente des histogrammes indiquant la production de VEGF par les cellules endothéliales humaines HSkMEC, par des cellules HUVEC immortalisées (iHUVEC), par des cellules FSkMEC et par des cellules endothéliales félines du cordon ombilical (« Feline Umbilical cord Endothelial Cells » :(FOmEC)) après infection par différentes souches de *Bartonella.* L'ordonnée représente la quantité de VEGF mesurée en picogrammes par millilitres (pg/ml).

### EXEMPLES

### Exemple 1 : Isolement et caractérisation de cellules endothéliales félines

### A. Matériel et méthodes

### A.1. Échantillons utilisés

Quatre embryons non viables et immatures ont été aseptiquement prélevés par césarienne d'une mère après 45 jours de gestation et 9 biopsies d'organes ou tissus ont été faites à partir de chaque embryon. Tous les tissus et organes ont été placés dans un milieu RPMI (marque de commerce) commercialisé par la société Gibco, BRL, Cergy-Pontoise France sans sérum foetal de bovin et supplémenté avec des antibiotiques (pénicilline et streptomycine) et stockés à 4°C.

### A.2. Isolement et culture des lignées cellulaires de cellules endothéliales félines

L'isolement des cellules endothéliales de la macrovascularisation, c'est à dire du cordon ombilical ou de la microvascularisation c'est à dire de la peau, des ganglions lymphatiques, de la rate, du cerveau, du foie de l'intestin et des plaques de Peyer a été faite selon les procédés décrits dans Bizouarne N, Denis V, Legrand A, Monsigny M, Kieda C: A SV-40 immortalized murine endothelial cell line from peripheral lymph node high endothelium expresses a new alpha-I-fucose binding protein. Biol Cell 1993, 79:209-218 **[27];** et dans Bizouarne N, Mitterrand M, Monsigny M, Kieda C: Characterization of membrane sugar specific receptors in cultured high endothelial cells from mouse peripheral lymph nodes.Biol Cell 1993, 79:27-35 **[28]**

### A.3. Immortalisation et sélection des lignées cellulaires de cellules endothéliales félines

Les cultures de cellules endothéliales ont été transfectées avec le néoplasmide pSV3-neoplasmid (ATCC) décrit dans Bizouarne et al. **[28].** Les cellules résistantes à la généticine ont été clonées et choisies selon le procédé décrit dans Bizouarne et al. **[27].**

Les lignées de cellules endothéliales félines ont été cultivées dans le milieu OptiMEM (marque de commerce) commercialisé par la société Invitrogen contenant du Gibco Glutamax (marque de commerce), 2 % de sérum bovin foetal, 5 % de fungizone (marque de commerce) et 0.4 % de gentamicine (marque de commerce). Les lignées ont été maintenues dans une atmosphère humide contenant 5 % de dioxyde de carbone (CO₂) à 37 °C.

### A.4. Caractérisation des lignées cellulaires de cellules endothéliales félines

### A.4.1. Corps intracellulaires de Weibel-Palade (« Weibel-Palade Bodies »WPB) et détection de l'enzyme de conversion de l'angiotensine

Toutes les lignées cellulaires établies ont été cultivées 48 heures sur des lames de microscope douze puits commercialisées par la société (ICN Bio-medicals,12 Aurora, OH, USA). Les monocouches ont été fixées pendant 10 minutes avec du paraformaldéhyde (PFA) commercialisé par la société Merck (Darmstadt (Allemagne)) à une concentration de 10 mg/ml et perméabilisées pendant 30 min à 37°C, avec du Triton14 X100 à 0,05%.

Pour la détection des corps intracellulaires de Weibel-Palade, un anticorps polyclonal de lapin anti-facteur humain de von Willebrand commercialisé sous le numéro catalogue A0082 par la société Dako à une concentration de 20 µg/ml a été appliqué sur les cellules pendant 2 heures à température ambiante, c'est à dire 20°C et lavé deux fois avec du tampon phosphate salin (PBS « Phosphate Buffered Saline ») contenant 1% de sérum albumine bovine. L'anticorps secondaire était un anti-corps fluorescent anti-immunoglobuline de lapin, fait chez la chèvre et marqué par l'isothiocyanate de fluorescéine à une concentration de 20 µg/ml commercialisé par la société SBE, CliniSciences, Montrouge, France, avec lequel les cellules ont été incubées pendant 30 minutes à température ambiante.

Pour la détection de l'enzyme de conversion de l'angiotensine des anticorps polyclonaux de lapin anti-enzyme de conversion de l'angiotensine humaine commercialisés sous la référence sc-20791 par la société Santa Cruz Biotechnology, dilués au 1 :50 dans du tampon phosphate de sodium salin (PBS « Phosphate Buffered Saline ») contenant 1% de sérum-albumine bovin, et un second anticorps de chèvre fluorescent anti immoglobuline de lapin marqué par l'isothiocyanate de fluorescéine a été utilisé. Les inventeurs ont utilisé des cellules épithéliales de foie de singes Vero line (ATCC, CCL-81) comme contrôle négatif. Les cellules ont été examinées avec un microscope à fluorescence Leica DMI 4000 B.

### A.5.2. essai d'angiogenèse

Une plaque 96 puits de la société Falcon, BD Biosciences, Grenoble, France a été recouverte avec du Matrigel (marque de commerce) commercialisé par BD Biosciences, Grenoble France pour mimer la matrice extracellulaire à raison de 40 µl par puits. Le Matrigel (marque de commerce) a été laissé pour polymérisation pendant 1 heure à 37 °C avant ensemencement des cellules à raison de 8 x 10³ cellules par puits. Le réarrangement et la formation de structures de type capillaires ont été observés et photographiés régulièrement à différents temps sous un microscope (commercialisé par Nikon TMS, Japon). Les cellules épithéliales de singes Vero line, ATCC, CCL-81 servent de contrôle négatif.

### B. Résultats

Sur la base de leur origine tissulaire, neuf lignées cellulaires endothéliales ont été établies, huit cellules à partir de la microvascularisation désignées comme cellules endothéliales félines de la peau (Feline Skin Microvascular Endothelial Cells : FSkMEC), cellules endothéliales félines de ganglions lymphatiques périphériques (Feline Peripheral Lymph Node Microvascular Endothelial Cells : FPLNMEC), cellules endothéliales félines Microvasculaires de poumons (Feline Lung Microvascular Endothelial Cells : FLuMEC), cellules endothéliales félines microvasculaires de cerveau (Feline Brain Microvascular Endothelial Cells : FBrMEC), cellules endothéliales félines microvasculaires de foie (Feline Liver Microvascular Endothelial Cells :FLiMEC), cellules endothéliales félines microvasculaires des plaques de Peyer (Feline Peyer's patches Microvascular Endothelial Cells : FPPMEC), cellules endothéliales félines microvasculaires de l'intestin (Feline Intestine Microvascular Endothelial Cells : FIntMEC), cellules endothéliales félines microvasculaires du coeur (Feline Heart Microvascular Endothelial Cells FHeMEC) et une lignée à partir de la macrovascularisation désignée cellules endothéliales félines du cordon ombilical (Feline Umbilical cord Endothelial Cells :FOmEC).

La présence de marqueurs endothéliaux : von Willebrand factor, vWf et l'enzyme de conversion de l'angiotensine (Angiotensin Converting Enzyme : ACE) a été confirmée par microscopie à fluorescence. Les cellules perméabilisées et traitées avec un anticorps anti-vWf antibody a révélé la présence de granules intracytoplasmiques de Weibel-Palade. Les granules ont été localisées autour du noyau et dispersées dans le cytoplasme. En outre, chaque lignée cellulaire féline testée était positive pour l'enzyme de conversion de l'angiotensine. Sa localisation était intracellulaire et sur la surface de la cellule (données non fournies).

Une des caractéristiques fonctionnelles des cellules endothéliales étant de soutenir l'angiogenèse, les lignées cellulaires ont été testées pour leur capacité à produire des structures de type capillaires dans un test classique Matrigel (marque de commerce). De manière aussi efficace que les cellules endothéliales humaines, les neuf lignées cellulaires établies ont été capables de réaliser une angiogenèse sur Matrigel. La figure 1 correspond à des photographies illustrant cette propriété.

Parmi les neuf lignées cellulaires endothéliales établies, les cellules endothéliales félines de la peau (FSkMEC) dérivées de la microvascularisation et les cellules endothéliales félines du cordon ombilical (FUcEC) dérivées de la macrovascularisation ont été choisies pour les expériences suivantes.

Cet exemple démontre clairement l'isolement, la caractérisation et l'établissement de lignées établies de cellules endothéliales félines.

### Exemple 2 : Etude comparative entre des cellules endothéliales humaines et des cellules endothéliales félines.

### A. Matériel et Méthode

### A. 1. Lignées cellulaires de cellules endothéliales humaines

Deux lignées cellulaires de cellules endothéliales organospécifiques décrites dans Kieda C, Paprocka M, Krawczenko A, Zalecki P, Dupuis P, Monsigny 1 M, Radzikowski C Dus D: New human microvascular endothelial cell lines with specific adhesion molecules phenotypes. Endothelium 2002, 9:247-261 **[29]** ont été utilisées. Une isolée de la macrovascularisation : les cellules endothéliales humaines de la veine ombilicale (HUVEC : « Human umbilical vein endothelial cell ») et une isolée de la microvascularisation de la peau : les cellules endothéliales humaines microvasculaire de la peau humaine (HSkMEC : « human skin endothelial cells »).

### A. 2. Lignées cellulaires de cellules endothéliales félines

Les lignées cellulaires de cellules endothéliales félines de la peau (FSkMEC) dérivées de la microvascularisation et les cellules endothéliales félines du cordon ombilical (FOmEC) dérivées de la macrovascularisation de l'exemple 1 ont été utilisées.

### A. 3. Souches Bactériennes

Quatre souches de *B. henselae* ont été utilisées sur la base de l'espèce d'origine : humain ou félin, et du génotype : I ou II. Les deux souches isolées à partir de patients humains étaient les souches Houston-1 (« strain Houston-1 » (génotype I (H1) / ATCC 49882)) et la souche génotype II Marseille (H2) fourni par Jean Marc Rolain (Unité des rickettsies, Marseille France). Les deux souches isolées à partir de chats étaient une souche de génotype I (F1/ Strain 297172) fourni par Bruno Chomel (University of California, Davis, USA) et une souche *B. henselae* génotype II isolé initialement par les inventeurs selon le procédé décrit dans Gurfield AN, Boulouis HJ, Chomel BB, Kasten RW, Heller R, Bouillin C, Gandoin C, Thibault D, Chang CC, Barrat F, Piemont Y. Epidemiology of Bartonella infection in domestic cats in France. Vet Microbiol. 2001 May 21;80(2):185-98 **[30].**

*B. tribocorum* (Bt) isolé de rats et sans pathogénicité connue pour l'humain et les chats a été utilisé comme contrôle.

Toutes les souches ont été cultivées sur un milieu agar additionné de 5% de sang de mouton commercialisé par la société BioMerieux, Craponne, France, pendant 5 à 7 jours dans une atmosphère humidifiée à 37 °C et 5 % CO₂.

### A.4 Analyse de l'expression de BadA dans les souches Bartonella

Les inventeurs ont vérifié la présence de pili dans les souches utilisées par détection de l'antigène de BadA via un procédé immunologique. Après 6 jours de croissance bactérienne sur un milieu agar au sang de mouton commercialisé par la société BioMerieux, Craponne-France, les souches *Bartonella* ont été récupérées et resuspendues dans du PBS. Les bactéries ont été utilisées à une concentration de 2 x 10⁸ par ml, qui a été déterminée par mesure de la densité optique à 600nm, une densité égale à 2 indiquant une concentration de 10⁹ bactéries par mL selon le protocole décrit dans Riess T, Raddatz G, Linke D, Schäfer A, Kempf VAJ: Analysis of Bartonella adhesin A expression reveals differences between various B. henselae strains. Infect Immun 2007, 75:35-43 [16]

Un millilitre de chacune des suspensions a été introduit dans un tube en plastique et incubé à 37°C avec 5% de CO₂ dans une atmosphère humide. Après 60 minutes, 10 µL de la suspension ont été prélevés du fond de chaque tube et transférés sur une lame de verre et fixés avec 4% de PBS-PFA. Après lavage avec du PBS, les sites non spécifiques ont été bloqués avec 0.2 % de BSA durant 20 minutes. Les bactéries ont été colorées avec un anticorps polyclonal de lapin anti Bad A sérum fourni par V. Kempf, Klinikum des Johann Wolfgang Goethe Universt¨t, Frankfurt am Main Allemagne, pendant 1 heure. Un anticorps secondaire, anticorps de chèvre anti-immunoglobuline de lapin marqué avec l'isothiocyanate de fluorescéine commercialisé par la société SBE CliniSciences, Montrouge, France, a été ajouté. Les bactéries ont été lavées avec du PBS et la détection a été faite avec un microscope à fluorescence Leica DMI 4000 B.

L'acide désoxyribonucléique (ADN) a été coloré avec du 4'-6 diamidino-2-phénylindole dihydrochlorure (DAPI) commercialisé sous la référence catalogue ref H-1200 par la société AbCys, France comme contrôle interne.

### A.5 Génération de surnageants de culture de Bartonella

Les souches de *B. henselae* et *B. tribocorum* ont été récupérées à partir des plaques d'agar contenant du sang et suspendue dans un milieu Schneider commercialisé par la société Gibco, France. Les cultures bactériennes ont été agitées pendant 18 heures à 200 tours par minutes (rpm : rotation par minute) et à 37°C sur un agitateur orbital. Après 18 heures d'incubation, les suspensions ont été prélevées des flacons et centrifugées à 2000 rpm pendant 10 minutes pour former un culot. Les surnageants sans bactéries ont été concentrés et diafiltrés avec de l'OptiMEM à l'aide d'un filtre centrifuge Amicon Ultra-15 MILLIPORE (marque enregistrée). Le surnageant de culture a été obtenu par le même procédé. Les concentrations protéiques ont été déterminées par test à l'acide bis inchonitique avec le kit BC Assay (marque de commerce) commercialisé par la société Uptima, Interchim.

### A.6. Induction in-vitro de l'angiogenèse

Vingt heures avant l'infection des cellules endothéliales HUVEC, HSkMEC, FOmEC et FSkMEC, les antibiotiques ont été éliminés du milieu de culture. Les cellules endothéliales, 8 x 10³ cellules par puits ont été infectées avec *Bartonella* à une multiplicité d'infection (« multiplicity of infection » MOI) de 50, 100 et 150 bactéries par cellules ou traitées avec du surnageant de culture à 250 µg/ml. Elles ont été ensuite ensemencées dans des plaques 96 puits préalablement recouvertes avec 40 µl par puits de Matrigel (marque de commerce) (BD Biosciences, Grenoble, France). Le réarrangement des cellules endothéliales et la formation de structures de type capillaire ont été acquis et enregistrés pour analyse d'image par le programme Zeiss axiovision. Le vidéo microscope Zeiss axiovert 200M équipé pour les contrôles de la température, du gaz et de l'humidité a été utilisé afin d'acquérir les images dans des intervalles de temps et reconstituer les cinétiques du procédé dynamique de l'angiogenèse. La formation de pseudo-vaisseaux a été suivie pendant 7 jours. La formation de structures de type capillaire a été quantifiée par mesure de la longueur des tubes et du nombre de points d'embranchement.

### A.7. Test de cicatrisation

Les cellules HUVEC, HSkMEC, FOmEC et FSkMEC ont été ensemencées dans une plaque 24 puits a une densité de 10⁵ cellules par puits et laissé dans un milieu OptiMEM sans antibiotiques pendant une nuit. Quand les cellules sont devenues confluentes, une blessure rectiligne a été faite dans le centre du puits en utilisant l'extrémité d'un cône de pipette stérile. Les cellules endothéliales ont été infectées par des bactéries à une MOI de 100 pendant 24 heures. La cicatrisation a été observée et photographiée sous un microscope Nikon TMS (marque de commerce). Les distances de cicatrisation (dans 21 µm) ont été mesurées aux temps 0 et à 24 heures après la blessure.

### A.8 Détermination du niveau intracellulaire d'AMPc

Après infection des cellules endothéliales HUVEC, HSkMEC FOmEC et FSkMEC par les souches *Bartonella* à une MOI 150 pendant 30 heures dans une plaque 24 puits, les cellules ont été lavées avec du PBS et lysées. Les niveaux intracellulaires d'AMPc ont été déterminés par le système EIA (marque de commerce) commercialisé par la société Biotrak, Amersham, Biosciences selon les préconisations du fabricant.

### A.9. Analyse de VEGFR-2 par buvardage de Western (Western blot analysis)

Après 24 heures d'infection des cellules endothéliales HUVEC, HSkMEC, FOmEC et FSkMEC par les diverses bactéries à une MOI de 100, les cellules HUVEC, HSkMEC, FOmEC et FSkMEC ont été lysées dans du tampon de lyse TNT comprenant 10 mM de Tris-HCL pH 7,5; 150 mM NaCL; 1% Nonidet P-40; 1% Triton X100 et 2mM d'EDTA complémenté avec des inhibiteurs de protéases et de phosphatases à savoir 10 µg/ml de leupeptine et aprotinine, 1 mM de fluorure de phénylméthylsulfonyle; 25 mM de NaF et 300 µM de vanadate, pendant 15 minutes à 4°C. Des quantités égales d'échantillons ont été soumises à une électrophorèse sur gel de polyacrylamide contenant du SDS et transférées sur une membrane de difluorure de polyvinylidène.

Les membranes ont été incubées avec un anticorps anti-VEGF-R2 et anti VEGFR-2 phosphorylé Y1175 commercialisés par la société Cell signaling, Ozyme, Saint-Quentin-en-Yvelines, France. Les anticorps ont été ensuite révélés en utilisant le colorant fluorescent dylight680 fluorescent commercialisé par la société Pierce Biotechnology conjugué avec des anticorps secondaires et les membranes ont été scannées et analysées avec le système d'imagerie infra-rouge Odyssey (marque de commerce) commercialisé par la société Li-Cor BioSciences, EuroSep, Cergy-Pontoise, France.

### B. Résultats

### B.1. Détermination de l'expression de BadA dans les souches B. henselae et B. tribocorum

BadA a été caractérisée comme étant un facteur pathogénique important pour *B. henselae* et doit être évaluée avant les expériences d'infection avec *B. henselae.* Trois souches *B.henselae* (H1, F1 et F2) expriment BadA, tandis que *B*. *henselae(H2)* et *B. tribocorum* ne l'expriment pas. La figure 2, qui représente l'expression de Bad-A, démontre clairement ces résultats : ligne BadA colonne H1, F1 et F2. De plus, seulement les souches positives pour BadA, H1, F1 et F2, s'autoagglutinent, tandis que les souches n'exprimant pas BadA : H2 et Bt, n'étaient pas auto-agglutinantes comme le montre la figure 2.

### B.2. Réponse angiogénique des cellules endothéliales félines et humaines à Bartonella

La capacité des lignées cellulaires humaines et félines non infectées à former des structures de type capillaires a été en premier évaluée par culture sur Matrigel (marque de commerce). Les inventeurs ont démontré que les structures de type capillaire des cellules endothéliales félines non infectées sont apparues rapidement dans les deux heures après ensemencement alors qu'avec les cellules endothéliales humaines non infectées, dérivées de la macrovascularisation (HUVEC) ces structures apparaissent environ dans les 5 heures et dans les 10 heures avec les cellules endothéliales dérivées de la microvascularisation (HSkMEC).

Au contraire des cellules endothéliales félines le réseau de pseudo-vaisseaux persiste plus longtemps avec les cellules endothéliales humaines. En effet, le réseau persiste jusqu'à 24 heures pour les cellules endothéliales humaines dérivées de la macrovascularisation et jusqu'à 7 jours pour les cellules endothéliales humaines de la microvascularisation, tandis que pour les cellules endothéliales félines ce réseau disparaît après 20 heures

Ces résultats démontrent donc clairement que les caractéristiques cellulaires des cellules endothéliales félines sont différentes de celles des cellules endothéliales humaines.

### B.3. Etude de l'infection par Bartonella de lignées cellulaires de cellules endothéliales félines et de lignées cellulaires de cellules endothéliales humaines

Les inventeurs ont également observé les effets d'une infection par *Bartonella* sur quatre lignées cellulaires à savoir les cellules endothéliales félines de la peau (FSkMEC) dérivées de la microvascularisation et les cellules endothéliales félines du cordon ombilical (FOmEC) dérivées de la macrovascularisation, les cellules endothéliales humaines dérivées de la macrovascularisation (HUVEC) et les cellules endothéliales humaines dérivées de la microvascularisation (HSkMEC)

Pour les cellules HSkMEC, indépendamment de l'origine des espèces et des génotypes de *Bartonella* utilisés, la formation du réseau de type capillaire était dépendante de la dose. Plus la MOI est grande plus rapide est la formation de tubes capillaires par rapport aux cellules HSkMEC non infectées. Les infections avec des MOI plus faibles, de 50 et 100 ont également accélérées la formation de tubes capillaires par rapport aux cellules non infectées comme le montre la figure 3A HSkMEC - H1 MOI 50, et HSkMEC - H1 MOI 100.

De plus, les cellules HSkMEC infectées par *Bartonella* présentent une morphologie allongée comparativement aux cellules du contrôle tel que présenté sur la figure 3A. Enfin les cellules HSkMEC infectées par *Bartonella* voient la densité des structures de type capillaires augmenter d'environ 2 fois comparativement aux cellules non infectées. La figure 3B montre clairement cette augmentation. Le réseau a été observé jusqu'à 7 jours avec ces cellules et a ensuite disparu.

Pour les cellules HUVEC, l'augmentation de la densité des structures de type capillaires est d'environ 2 fois dans les cellules infectées comparativement aux cellules non infectées. Cependant, cette formation n'était pas plus précoce. De plus, ce réseau a été observé seulement jusqu'à 24 heures avant de disparaître.

Aucune différence visible n'a été observée sur les cinétiques de formation de structure de type capillaire quand les cellules FOmEC et FSkMEC ont été mises en culture sur du Matrigel (marque de commerce) et ceci indépendamment de l'origine de l'hôte et du génotype de *Bartonella* utilisé.

Cependant, 2 heures après ensemencement, l'angiogenèse de FOmEC et FSkMEC avait déjà commencé. En outre, la destruction du réseau est plus rapide dans les lignées cellulaires infectées comparativement aux lignées cellulaires non infectées (résultats non fournis). Ainsi, l'effet de *Bartonella* sur la formation du réseau était plus importante sur les cellules endothéliales humaines que sur les cellules endothéliales félines et dépendant de la dose. En outre, l'effet était plus persistant sur les cellules endothéliales humaines de la microvascularisation par rapport à la macrovascularisation.

Ces résultats démontrent clairement que les cellules endothéliales félines et humaines ne réagissent pas de la même manière à une infection par un microorganisme pathogène.

Ces résultats démontrent également clairement que les cellules de l'invention peuvent être utilisées comme modèle cellulaire pour suivre et/ou démontrer une spécificité d'espèce de maladies infectieuses.

Ces résultats démontrent également clairement que les cellules de l'invention peuvent être utilisées pour démontrer et étudier la spécificité et le ciblage d'un type endothélial par rapport à l'autre en terme de caractéristiques vasculaires.

### B.4. Réponse angiogénique des cellules endothéliales aux surnageants de culture de Bartonella

Afin d'étudier les effets du sécrétome de *Bartonella,* différents surnageants de culture de *Bartonella* ont été générés et testés pour leur capacité à induire l'angiogenèse de cellules endothéliales félines et humaines. A une concentration protéique de 250 µg /mL, les surnageants de culture de *Bartonella Bartonella* ont induit une formation de tubes capillaires chez les cellules endothéliales humaines comme représentée sur la figure 3A).

Les réponses angiogéniques obtenues avec les surnageants de culture de *Bartonella* ont été accélérées 2 fois en comparaison avec les cellules contrôles, comme lors d'une infection des cellules par *Bartonella* à une MOI de 150 (figures 3A and 3B).

La morphologie des cellules endothéliales humaines incubées avec le surnageant de culture était similaire à celle des cellules infectées par *B*. *henselae* aux MOIs de 50, 100 ou 150 comme le montre la figure 3A.

Par ailleurs, le surnageant de culture n'a pas accéléré l'angiogenèse des cellules endothéliales félines.

Ces résultats démontrent clairement que les cellules de l'invention peuvent être utilisées notamment pour cribler des molécules permettant ainsi de déterminer si ces molécules ont un effet sur les cellules, par exemple si elles ont un effet sur l'angiogenèse.

Ces résultats démontrent également clairement que l'on peut déchiffrer les mécanismes de l'activité d'un pathogène en utilisant les cellules de l'invention, que les cellules de l'invention peuvent être utilisées pour démontrer la spécificité d'espèce de l'activité d'un pathogène et/ou que l'on peut déterminer le mécanisme moléculaire de l'action du microorganisme à l'aide des cellules de l'invention.

### B.5. Etude de l'effet de l'infection par Bartonella sur la migration/ cicatrisation des cellules endothéliales induite par une plaie.

La migration des cellules endothéliales est une étape essentielle pour l'angiogenèse. Dans cette expérience, l'importance du site de l'infection concernant la susceptibilité de l'endothélium a été examinée.

La réaction des cellules endothéliales selon leur origine tissulaire a été observée après infection en mesurant leur vitesse de migration pour cicatriser une plaie mécanique. Non infectées, les cellules HUVEC la cicatrisent au moins 5 fois plus rapidement que les cellules HSkMEC mais l'infection induit une accélération de la cicatrisation des cellules HUVEC et HSkMEC. En outre, la vitesse de guérison des cellules HUVEC était approximativement doublée tandis qu'elle augmente d'un facteur 4 à 7 pour les cellules HSkMEC (figure 4).

La migration induite par la blessure a été également observée pour les cellules endothéliales félines FOmEC et FSkMEC mais pas de stimulation par infection par *Bartonella.*

Ces résultats démontrent clairement que les cellules de l'invention sont utiles pour la vérification de la validité biologique de l'observation de la spécificité d'espèce et pour la détermination de l'effet de molécules pouvant agir sur des agents infectieux et/ou microorganismes.

### B.6. Effet de l'infection par Bartonella sur les cellules endothéliales humaines et félines sur la production d'AMPc

Le niveau d'AMPc induit par l'infection de quatre lignées cellulaires endothéliales par différentes *Bartonella,* a montré que l'infection par la souche de génotype I de *B. henselae* de cellules endothéliales HUVEC a déclenché la production d'AMPc.

Chez les cellules endothéliales félines de la macrovascularisation et microvascularisation, le taux d'AMPc produit par les cellules félines FUcEC et FSkMEC était inférieur au taux d'AMPc produit par les cellules endothéliales humaines HUVEC et HSkMEC

Le taux produit par la cellule HUVEC macrovasculaire était supérieur par rapport à la production de cellules de la microvascularisation (HSkMEC). Pour les cellules endothéliales félines, il n'a pas été observé de différence entre les cellules endothéliales de la macrovascularisation et de la microvascularisation quant à la production très faible d'AMPc. Ces résultats sont clairement décrits sur la figure 5.

Dans les cellules endothéliales infectées, les quantités d'AMPc produit par les cellules humaines et félines de la macrovascularisation étaient supérieures à celles produites par les cellules de la microvascularisation.

Les cellules HUVEC infectées par la souche H1 (humaine) ont produit un niveau supérieur d'AMPc. Cependant, l'infection de cellules HUVEC par d'autres souches telles que les souches félines F1, F2 et la souche Bt n'ont pas induit une telle production d'AMPc.

L'infection des cellules endothéliales humaines et félines de la microvascularisation a induit une faible élévation de l'AMPc indiquant que le procédé d'activation de la signalisation AMPc par l'infection bactérienne est dépendant de l'espèce et organe dépendant.

Ces résultats démontrent clairement que les cellules de l'invention permettent donc d'étudier et de disséquer les mécanismes d'infection en fonction de l'organe, du type vasculaire et de l'espèce.

### B.7. Effets de l'infection par B. henselae sur l'activation de VEGFR-2

Le facteur de croissance vasculaire endothéliale (vascular endothelial growth factor: VEGF) est le principal facteur angiogénique régulant de façon positive la migration, la prolifération et la survie des cellules endothéliales. L'induction de la production de VEGF par les macrophages par l'infection par *Bartonella* a été suggérée dans l'état de la technique *in vivo* et *in vitro.*

Cet exemple étudie le niveau d'activation du récepteur du VEGF, le VEGFR-2 qui est impliqué dans les effets pro angiogéniques.

Les inventeurs ont montré que l'infection par *Bartonella* des cellules endothéliales HUVEC, HSkMEC, FOmEC et FSkEC était associée avec une augmentation de la phosphorylation du VEGFR-2 (figure 6).

De plus, les inventeurs ont montré que le niveau de VEGFR-2 phosphorylé est supérieur quand les cellules sont infectées avec une souche provenant d'un hôte homologue, c'est-à-dire quand les cellules endothéliales humaines sont infectées par des souches originellement infectant l'humain et isolées de l'humain et quand les cellules endothéliales félines sont infectées avec des souches originellement infectant le chat et isolées du chat.

Cet exemple démontre clairement qu'il existe un mécanisme liant l'espèce des cellules endothéliales avec l'infection bactérienne et que l'invention peut servir à démontrer cette spécificité d'espèce d'un pathogène.

### C. Discussion

*B. henselae* est un pathogène intracellulaire facultatif associé avec l'induction des tumeurs vasoprolifératives chez les hommes mais pas chez les chats.

La stimulation angiogénique par l'infection par *B*. *henselae* représente une caractéristique de la pathogénécité bactérienne et représente un intérêt particulier pour la compréhension de la formation de tumeurs provoquées par les bactéries. Cependant seulement peu de données contradictoires ont été obtenues concernant les mécanismes impliqués dans les maladies angiomateuses induites par *B*. *henselae.* Conley et *al,* [18] ont montré que le facteur stimulant de *B*. *henselae* est une protéine localisée dans une fraction particulaire tandis que d'autres ont fourni des preuves de facteurs solubles sécrétés par les bactéries

Les exemples démontrent clairement la nécessité d'utiliser des cellules endothéliales organospécifiques pour étudier les interactions entre *B. henselae* et les cellules endothéliales. Par ailleurs il est important de comparer les résultats obtenus avec les cellules humaines à ceux fournis par les cellules endothéliales félines. Dans cet exemple, il est clairement démontré une différence de réactivité entre les cellules humaines et les cellules félines et entre les cellules de la macrovascularisation et de la microvascularisation.

Cet exemple démontre également clairement la nécessité d'avoir des lignées cellulaires établies de cellules endothéliales félines et également d'avoir des lignées cellulaires établies de cellules organospécifiques de la microvascularisations et de la macrovascularisations.

Les cellules de l'invention permettent donc d'étudier et de disséquer les mécanismes d'infections en fonction de l'organe, du type vasculaire et de l'espèce.

De plus, il est nécessaire de considérer lors d'une infection les conditions physiologiques locales : le microenviroennement. Pour ce faire et comme le démontre clairement cet exemple les cellules de l'invention constituent un nouvel outil très utile notamment pour étudier les facteurs cellulaires et bactériens déterminant la réactivité des microorganismes pathogènes chez les hommes par rapport au félins, comment il provoque, par exemple des mécanismes antiapoptotiques et/ou proangiogéliques résultant de l'angiomatose bacillaire et de la péliose uniquement chez les humains.

Par ailleurs, les résultats obtenus montrent que les effets de sur les cellules dérivées de la microvascularisation ne peuvent pas être représentés de façon complète par les cellules dérivées de la macrovascularisation.

En outre, les résultats obtenus ont permis de mettre en évidence les différences intrinsèques dans les cinétiques angiogéniques et dans la cicatrisation entre les cellules endothéliales humaines dérivées de la microvaculature et macrovascularisation

La réponse endothéliale à une infection par *Bartonella* semble agir à deux niveaux du procédé angiogénique. L'accélération du réarrangement des cellules a été observée seulement avec les cellules endothéliales humaines dérivées de la microvascularisation tandis que la quantité de pseudo-vaisseaux a été augmentée avec les cellules endothéliales humaine microvasculaires et macrovasculaires.

Les résultats montrent que les cellules endothéliales félines macrovasculaires et microvasculaires sont différentes. En effet, l'infection par *B*. *henselae* n'a pas d'effet visible proangiogénique in-vitro mais accélère la destruction du réseau de cellules félines. Ces résultats récapitulent la situation clinique, c'est-à-dire l'absence de lésions pseudotumorales chez les chats alors que chez les hommes BA.

Les cellules de l'invention constituent donc un nouvel outil très utile notamment pour comprendre les bases moléculaire et cellulaires du potentiel zoonotic de *B. henselae* et d'explorer les mécanismes moléculaires et cellulaires sous-jacent de BA/BP.

De plus, les exemples ci-dessus démontrent que bien que *B*. *henselae* provoque la prolifération *in vitro* des cellules HUVEC, la prolifération est inhibée à des MOI supérieures due à l'effet cytotoxique du système de sécrétion de type quatre encodé par virB.45. Cependant, la prolifération endothéliale est uniquement une partie de l'angiogenèse qui comprend également la migration des cellules endothéliales, la survie des cellules dans de nouveaux types de matrices extracellulaires, la différenciation et l'organisation en réseaux de vaisseaux. Pour les cellules HSkMEC, l'augmentation de la formation de structures de type capillaires et la précocité de la formation capillaire a été augmentée avec des grandes MOI.

Aucun effet cytotoxique de VirB n'a été exercé durant les expériences de formation d'angiogenèse, même à la plus forte dose utilisée, comme cela était suggéré dans l'état de la technique pour les cellules HUVEC.

Le modèle d'infection basé sur des cellules endothéliales humaines de la microvascularisation tend également à montrer que, indépendamment de l'espèce de la bactérie *B. henselae ou B. tribocorum,* ou du génotype (I ou II) de *B. henselae,* toutes les souches de *Bartonella* testées peuvent induire *in vitro* les mêmes effets angiogéniques.

Les résultats ont montré que pour l'accélération du procédé angiogénique *in vitro,* un contact direct de la bactérie avec les cellules endothéliales n'était pas nécessaire. Aussi la bactérie *B. henselae* doit produire et sécréter des facteurs de croissance endothéliale.

Les études précédentes dédiées aux interactions entre les cellules HUVEC et *B. henselae* de génotype I ont mis en évidence la production d'AMPc dans l'activité de *B. henselae.*

Les cellules HSkMEC ne produisent pas, contrairement aux cellules HUVEC de niveau élevé d'AMPc. Ces résultats confirment donc les résultats obtenus précédemment avec les cellules HUVEC. En effet, il faut tenir compte des cellules HUVEC qui sont des cellules dérivées de la macrovascularisation, et donc très différentes des cellules endothéliales de la microvascularisation. Les cellules HSkMEC représentent un modèle de cellule endothéliale microvasculaire de la peau impliqué cliniquement dans l'angiomatose et la péliose bacillaire.

Tel que le montrent les résultats obtenus, les taux d'AMPc produits par les cellules endothéliales félines microvasculaires ou macrovasculaires étaient toujours significativement inférieurs à ceux produits par les cellules humaines indépendamment de l'infection indiquant une différence de réactivité entre les deux espèces.

Enfin, les résultats démontrent également l'activation qui se traduit par la phosphorylation du récepteur 2 du VEGF (VEGFR-2). Celui-ci n'est toutefois phosphorylé lors de l'infection par des souches homologues. Il est connu que le VEGF est le facteur clé de l'angiogenèse. Aussi, l'augmentation de la phosphorylation du VEGFR-2 et donc son activation lors de l'infection par des souches homologues peut refléter l'existence d'un contrôle adaptatif de *B. henselae* aux différentes espèces hôtes et donc contribuer à sa susceptibilité chez l'homme. L'activation de VEGFR-2 par *Bartonella* montre que le VEGF s'est fixé à son récepteur et que le signal d'activation s'est normalement exprimé par la phosphorylation de la molécule. Par ailleurs, il a été montré dans l'état de la technique que des patients avec BA ou BP ; ou avec un verruga peruviana induit par *Bartonella bacilliformis* présentaient un fort taux de VEGF ou une forte expression des récepteurs 1 et 2 du VEGF par les cellules endothéliales. Il est donc fortement probable que le VEGF soit un des facteurs impliqué.

Tel que le démontrent les résultats, les cellules de l'invention permettent donc également d'identifier des molécules impliquées dans des mécanismes cellulaires.

Cet exemple démontre donc clairement par les résultats obtenus pour l'angiogenèse *in vitro,* la stimulation de la guérison des plaies, l'induction de l'AMPc et l'activation du VEGFR-2, l'importance de l'origine tissulaire des cellules endothéliales et la spécificité de la relation entre les diverses souches de *B. henselae* et des lignées cellulaires humaines *versus* lignées cellulaires félines.

### Exemple 3 : Criblage de molécules permettant l'identification de molécule susceptibles d'induire, ou d'inhiber l'angiogenèse de cellules endothéliales félines

Les cellules utilisées dans cet exemple sont celles isolées et caractérisées dans l'exemple 1 précité.

Le milieu de culture utilisé est le milieu OptiMEM (marque de commerce) commercialisé par la société Invitrogen contenant du Gibco Glutamax (marque de commerce), 2 % de sérum foetal de veau, 5 % de fungizone (marque de commerce) et 0.4 % de gentamicine (marque de commerce).

Les différentes cellules sont cultivées indépendamment dans différents puits pendant 24 heures dans une atmosphère humide contenant 5 % de dioxyde de carbone (CO₂) à 37°C.

Ensuite, la molécule à tester est introduite dans le milieu de culture. Un témoin sans la molécule est réalisé dans les mêmes conditions et correspond au contrôle négatif. Les cellules sont mises en culture pendant 24 heures à 37°C.

L'effet de la molécule sur l'angiogenèse des cellules endothéliales est observé au microscope à différents temps selon le protocole décrit dans l'exemple 2.

La molécule testée stimule l'angiogenèse si l'apparition de structures de type capillaire est accélérée par rapport au contrôle négatif. Au contraire si l'apparition de structure de type capillaire est ralentie, voir inexistante par rapport au contrôle négatif, la molécule inhibe l'angiogenèse.

### Exemple 4: Criblage de molécules permettant l'identification de molécule susceptibles d'avoir une action sur un microorganisme pathogène

Les cellules utilisées dans cet exemple sont celles isolées et caractérisées dans l'exemple 1 précité.

Le milieu de culture utilisé est le milieu OptiMEM (marque de commerce) commercialisé par la société Invitrogen contenant du Gibco Glutamax (marque de commerce), 2 % de sérum foetal de veau, 5 % de fungizone (marque de commerce) et 0.4 % de gentamicine (marque de commerce).

Les différentes cellules sont cultivées indépendamment dans différents puits pendant 48 heures dans une atmosphère humide contenant 5 % de dioxyde de carbone (CO₂) à 37°C.

Les microorganismes utilisés sont les bactéries utilisées dans l'exemple 2, partie A.3. et cultivés selon le protocole décrit dans l'exemple 2 ou les microorganismes intracellulaires stricts cités dans l'exemple 5.

Vingt-quatre heures avant l'infection des cellules endothéliales, les antibiotiques sont extraits du milieu de culture. Les cellules endothéliales, à raison de 3x10⁴ cellules par puits sont infectées indépendamment avec une bactérie à une multiplicité d'infection (« multiplicity of infection » MOI) de 100 bactéries par cellules ou traitées avec du surnageant de culture bactérien ne contenant plus de bactéries et représentant le témoin négatif d'infection.

Après introduction des bactéries, la molécule à tester est introduite dans les milieux de culture à l'exception d'un milieu par cellule correspondant au témoin négatif.

Les cellules sont laissées en culture pendant 24 heures. Après élimination du milieu contenant l'antibiotique, les cellules sont récoltées et lysées par congélation décongélation et le lysat est mis en culture afin de déterminer la concentration bactérienne présente dans la cellule. Pour l'étude de l'effet de molécule susceptible d'agir sur des micro organismes intracellulaires stricts, l'évaluation de l'effet s'effectue par quantification des bactéries intracellulaires par Immunofluorescence ou par PCR quantitative. Si la concentration bactérienne est augmentée de manière identique à celle du témoin négatif, la molécule testée est sans effet, inversement, si la concentration bactérienne est diminuée ou nulle, la molécule testée est active sur la bactérie et permet en outre l'inhibition de sa croissance.

### Exemple 5 : Production de microorganismes spécifiques des cellules endothéliales félines.

Les cellules utilisées dans cet exemple sont celles isolées et caractérisées dans l'exemple 1 précité.

Le milieu de culture utilisé est le milieu OptiMEM (marque de commerce) commercialisé par la société Invitrogen contenant du Gibco Glutamax (marque de commerce), 2 % de sérum foetal de veau, 5 % de fungizone (marque de commerce) et 0.4 % de gentamicine (marque de commerce).

Les différentes cellules sont cultivées indépendamment dans différents tubes pendant 24 heures dans une atmosphère humide contenant 5 % de dioxyde de carbone (CO₂) à 37°C.

Les microorganismes utilisés sont les bactéries intracellulaires obligatoires *Mycoplasma harmofelis* et *Mycoplasma haemominutum, Rickettsia spp., Anaplasma phagocytophilum* qui sont des microorganismes spécifiques des cellules endothéliales et susceptibles de se reproduisent par infection des cellules endothéliales féline.

Vingt quatre heures avant l'infection des cellules endothéliales, les antibiotiques sont extraits du milieu de culture. Les microorganismes sont introduits dans chaque puits et les cellules sont mises en culture vingt quatre heures à 37°C (Mycoplasma *harmofelis* et *Mycoplasma haemominutum, Anaplasma phagocytophilum)* ou 30°C (*Rickettsia felis).*

Les microorganismes sont ensuite récupérés soit par lyse des cellules et récupération du lysat soit par récupération des cellules infectées entières

### Exemple 6 : Production de chimiokines spécifiques des cellules endothéliales félines

Les cellules de la présente invention permet de produire des facteurs solubles caractéristique de l'espèce féline et ceci de manière organospécifique. La production de la chémokine CCL21 à partir des cellules endothéliales de la microvascularisation de ganglions lymphatiques périphériques; la chémokine CXCL16 à partir des cellules endothéliales de la microvascularisation de la peau, ; la chémokine CCL28 (MEC) à partir des cellules endothéliales de la microvascularisation du poumon.

Les cellules sont cultivées dans le milieu de culture décrit dans l'exemple 1 précité et dans des conditions hypoxiques, c'est-à-dire dans un environnement dans lequel la valeur de la pO₂ est comprise entre 1 et 2%. Cette valeur de pO₂ provoque une activation spécifique des cellules endothéliales induisant ainsi la production des chémiokines.

### Exemple 7 : étude de l'effet d'infections de cellules endothéliales félines et humaines par des souches de Bartonnella sur la production du VEGF dans le milieu de culture.

Les cellules ont été cultivées dans le milieu de culture décrit dans l'exemple 1.

La mesure de la concentration de VEGF dans le milieu de culture a été effectuée via l'utilisation d'un Kit ELISA pour le VEGF humain commercialisé par la société R&D Systems, Minneapolis, MN, USA et mis en oeuvre selon les recommandations du fournisseur.

Les cellules ont été ensemencées à une densité de 5x10⁴ cellules/0,5ml/puits dans une plaque 24 puits et reposées pendant 12 heures. Ensuite, les cellules ont été infectées par différentes souches de *Bartonella* à une MOI de 100. Après 72 heures d'incubation, le milieu de culture a été récupéré. Le nombre de cellules a été déterminé immédiatement après la récupération des cellules en utilisant un Coulter counter.

Les différentes cellules utilisées étaient les cellules endothéliales humaines HSkMEC, des cellules HUVEC immortalisées (iHUVEC), des cellules FSkMEC et des cellules endothéliales félines du cordon ombilical (« Feline Umbilical cord Endothelial Cells » :(FOmEC)) telles que décrites précédemment. Pour chaque type de cellules, un million de cellules ont été testées.

Les différentes souches de Bartonellia utilisées étaient : la souche *B*. *henselae* de type I humaine (H1) (référence ATCC 49882), la souche *B*. *henselae* de type II humaine (H1), la souche *B*. *henselae* de type I féline (F1) (référence F1 297172), la souche *B. henselae* de type Il féline (F2) et la *B. tribocorum.*

La figure 7 représente les moyennes des mesures du VEGF obtenues par ELISA de milieux de culture issus des différentes cellules précitées infectées avec l'une des différentes souches précitées. Les résultats représentent la moyenne plus ou moins la déviation standard.

Comme représenté sur la figure 7, l'effet de l'infection sur la production du VEGF par les cellules est différent d'un type de cellules à un autre. La production du VEGF est augmentée dans les cellules humaines de la microvasculature : HskMEC après infection.

Ainsi, les inventeurs ont démontré l'implication du VEGF dans l'action de *B. henselae* à la lumière de l'origine tissulaire. Une production différentielle a été mise en avant entre les cellules endothéliales non infectée de la microvasculature et de la macrovasculature qu'elles soient d'origine humaine ou féline.

En outre, les quatre souches différentes de *B henselae* augmentent de manière significative la production de VEGF par les cellules HSkMEC (2 fois) tandis que la souche *B. tribocorum* augmente dans une moindre mesure cette production (1,3 fois).

De plus, la production du VEGF par les cellules félines n'est pas significativement affectée par l'infection avec les différentes souches (figure 7).

Enfin, les inventeurs ont obtenus des résultats similaires sur la production de l'interleukine 8 (IL8) par les cellules en fonction de leur origine tissulaire et/ou espèce (résultats non fournis)

Comme démontré dans cet exemple, les cellules de l'invention permettent donc d'étudier l'expression différentielle de facteurs significatifs de pathologies. En outre, les cellules de l'invention permettent d'étudier ces effets en fonction, par exemple de l'origine tissulaire des cellules.

En outre, les cellules de l'invention peuvent être utilisées, par exemple dans des tests d'inflammations et/ou des tests évaluant les effets possibles de composés, par exemple des anti-inflammatoires en fonction des différentes espèces et selon la pathologie.

### Liste des références

1. Regnery RL, Anderson BE, Clarridge JE, Rodriguez-Barradas MC, Jones DC, Carr JH: Characterization of a novel Rochalimaea species, R. henselae sp. nov., isolated from blood of a febrile, human immunodefeciency virus-positive patient. J Clin Microbiol 1992, 30:265-274
2. Boulouis HJ, Haddad N, Vayssier-Taussat M, Maillard R, Chomel B: Persistent Bartonella infection: epidemiological and clinical implications. Bull Acad Natl Med 2007, 191:1037-10 1044
3. Koehler JE, Sanchez MA, Garrido CS, Whitfeld MJ, Chen FM, Berger TG, Rodriguez- Barradas MC, Leboit PE, Tappero JW: Molecular epidemiology of Bartonella infections in patients with bacillary angiomatosis-peliosis. N Engl J Med 1997, 337:1876-1883
4. Boulouis HJ, Chang CC, Henn JB, Kasten RW, Chomel BB: Factors associated with the rapid emergence of zoonotic Bartonella infections. Vet Res 2005, 36:383-410
5. Yamamoto K, Chomel BB, Kasten RW, Hew CM, Weber DK, Lee WI: Experimental infection of specific pathogen free (SPF) cats with two different strains of Bartonella henselae type I: a comparative study. Vet Res 2002, 3:669-684
6. Breitschwerdt EB: Feline bartonellosis and cat scratch disease. Vet Immunol Immunopathol 2008, 123:167-71
7. Bergmans AM, Schellekens J, van Embden J, Schouls LM: Predominance 1 of two Bartonella henselae variants among cat-scratch disease patients in the Netherlands. J Clin Microbiol 1996, 34:254-260
8. Sander A, Ruess M, Deichmann K: Two different genotypes of Bartonella henselae in children with cat-scratch disease and their pet cats. Scand J Infect Dis 1998, 30:387-391
9. Sander A, Posselt M, Bohm N, Ruess M, Altwegg M: Detection of Bartonella henselae DNA by two different PCR assays and determination of the genotypes of strains involved in histologically defined cat scratch disease. J Clin Microbiol 1999, 37:993-997
10. Fournier PE, Robson J, Zeaiter Z, Mc Dougall R, Byrne S, Raoult D: Improved culture from lymph nodes of patients with cat scratch disease and genotypic characterization of Bartonella henselae isolates in Australia. J Clin Microbiol 2002, 40:3620-3624
11. Dillon B, Valenzuela J, Don R, Blanckenberg D, Wigney DI, Malik R, Morris AJ, Robson JM, Iredell J: Limited diversity among human isolates of Bartonella henselae. J Clin Microbiol 2002, 40:4691-4699
12. Woestyn S, Olivé N, Bigaignon G, Avesani V, Delmée M: Study of genotypes and virB4 secretion gene of Bartonella henselae strains from patients with clinically defined cat scratch disease. J Clin Microbiol 2004, 42:1420-1427
13. Bouchouicha R, Durand B, Monteil M, Chomel B, Berrich M, Birtles R, Breitschwerdt E, Koehler J, Kasten R, Petit E, Maruyama S, Arvand M, Boulouis H-J, Haddad N: Epidemiological applications of Multi-locus Variable number tandem 1 repeat Analysis (MLVA) for Bartonella henselae of Human and Feline origins. Emerg Infect Dis 2009, 15: 813-816
14. LeBoit PE, Berger TG, Egbert BM, Beckstead JH, Yen TSB, Stoler MH: Bacillary angiomatosis: The histopathology and differential diagnosis of a pseudoneoplastic infection in patients with human immunodeficiency virus disease. Am J Surg Pathol 1989, 13:909-920
15. Batterman HJ, Peek JA, Loutit JS, Falkow S, Tompkins LS: Bartonella henselae and Bartonella quintana adherence to and entry into cultured human epithelial cells. Infectlmmun 1995, 63:4553-4556
16. Riess T, Raddatz G, Linke D, Schäfer A, Kempf VAJ: Analysis of Bartonella adhesin A expression reveals differences between various B. henselae strains. Infect Immun 2007, 75:35-43
17. Riess T, Andersson SG, Lupas A, Schaller M, Schäfer A, Kyme P, Martin J, Wälzlein JH, Ehehalt U, Lindroos H, Schirle M, Nordheim A, Autenrieth IB, Kempf VA: Bartonella adhesin A mediates a proangiogenic host cell response. J Exp Med 2004, 200:1267-1278
18. Conley T, Slater L, Hamilton K: Rochalimaea species stimulate human endothelial cell proliferation and migration in vitro. J Lab Clin Med 1994, 124:521-528
19. Palmari J, Teysseire N, Dussert C, Raoult D: Image cytology and topographical 1 analysis of proliferation of endothelial cells in vitro during Bartonella (Rochalimaea) infection. Analytical Cell Pathol 1996, 11:13-30
20. Maeno NH, Oda K, Yoshiie MR, Wahid TF, Matayoshi S: Live Bartonella henselae enhances endothelial cell proliferation without direct contact. Microb Pathog 1999,7 27:419-427
21. McCord AM, Cuevas J, Anderson BE: Bartonella-induced endothelial cell proliferation is mediated by release of calcium from intracellular stores. DNA Cell Biol 2007, 26:657-11 663
22. McCord AM, Burgess AWO, Whaley MJ, Anderson BE: Interaction of Bartonella henselae with endothelial cells promotes monocyte/macrophage chemoattractant protein 1 gene expression and protein production and triggers monocyte migration. Infect Immun 2005, 73:5735-5742
23. Kempf VA, Volkmann B, Schaller M: Evidence of a leading role for VEGF in Bartonella henselae-induced endothelial cell proliferations. Cell Microbiol 2001, 3:623-632
24. Resto-Ruiz SI, Schmiederer M, Sweger D, Newton C, Klein TW, Friedman H, Anderson BE: Induction of a potential paracrine angiogenic loop between human THP-1 macrophages and human microvascular endothelial cells during Bartonella henselae infection. Infect Immun 2002, 70:4564-4570
25. Ferrara N, Gerber HP, Le Couter J: The biology of VEGF and its receptors. Nature Medicine 2003, 9:669-676
26. Schmid MC, Scheidegger F, Dehio M, Balmelle-Devaux N, Schulein R, Guye P, Chennakesava CS, Biedermann B, Dehio C: A translocated bacterial protein protects vascular endothelial cells from apoptosis. PLoS Pathog 2006, 2:1083-1096
27. Bizouarne N, Denis V, Legrand A, Monsigny M, Kieda C: A SV-40 immortalized murine endothelial cell line from peripheral lymph node high endothelium expresses a new alpha-l-fucose binding protein. Biol Cell 1993, 79:209-218
28. Bizouarne N, Mitterrand M, Monsigny M, Kieda C: Characterization of membrane sugar specific receptors in cultured high endothelial cells from mouse peripheral lymph nodes.Biol Cell 1993, 79:27-35
29. Kieda C, Paprocka M, Krawczenko A, Zalecki P, Dupuis P, Monsigny 1 M, Radzikowski C Dus D: New human microvascular endothelial cell lines with specific adhesion molecules phenotypes. Endothelium 2002, 9:247-261
30. Gurfield AN, Boulouis HJ, Chomel BB, Kasten RW, Heller R, Bouillin C, Gandoin C, Thibault D, Chang CC, Barrat F, Piemont Y. Epidemiology of Bartonella infection in domestic cats in France. Vet Microbiol. 2001 May 21;80(2):185-98
31. Fletcher NF, Brayden DJ, Brankin B, Worrall S, Callanan JJ Growth and characterisation of a cell culture model of the feline blood-brain barrier. Vet Immunol Immunopathol. 2006 Feb 15;109(3-4):233-44.

## Revendications

1. Cellule endothéliale féline organospécifique isolée **caractérisée en ce qu'**elle comprend le facteur de Von Willebrand, l'enzyme de conversion de l'angiotensine et les clusters de différentiation (CD) CD 31, 34, 105, 54, 62E, 62P, 146 choisie parmi les cellules déposées à la Collection Nationale de Culture de Microorganismes (CNCM), sous les numéros CNCM n°I-4250 (F Int MEC), n° I-4251 (F PLN MEC ), n° I-4252 (FOm EC ), n° I-4253 (F Li MEC )n° I-4254 (F Sk MEC ),n° I-4255 (F PP MEC ), n° I-4256 (F He MEC ), n° I-4257 (F Br MEC ), n° I-4258 (F Lu MEC).

2. Procédé de criblage de molécules susceptibles d'induire une différentiation et/ou une spécialisation d'une cellule isolée selon la revendication 1 comprenant les étapes suivantes :
- introduction d'au moins une molécule à cribler dans un milieu adapté à la culture de ladite cellule,
- introduction d'au moins une cellule isolée selon la revendication 1 dans ledit milieu,
- culture desdites cellules dans ledit milieu pendant un temps suffisant pour permettre la différenciation et/ou la spécialisation desdites cellules,
- prélèvement d'au moins une cellule cultivée dudit milieu de culture,
- observation de la différentiation et/ou spécialisation de ladite cellule par observation phénotypique de la cellule et/ou par révélation de marqueurs de différentiation.

3. Procédé selon la revendication 2, dans lequel la révélation de marqueurs de différentiation est effectuée au moyen d'anticorps spécifiques desdits marqueurs.

4. Procédé selon la revendication 3, dans lequel lesdites molécules sont susceptibles d'induire, ou d'inhiber une angiogenèse.

5. Procédé d'étude in vitro de pathologies dues à un microorganisme pathogène comprenant les étapes suivantes :
- culture d'au moins une cellule isolée selon la revendication 1 dans un milieu de culture approprié,
- inoculation de ladite cellule avec un microorganisme pathogène,
- culture des cellules inoculées, et
- observation d'un ou de plusieurs des critères suivants : évolution du phénotype des cellules, évolution de la cytotoxicité, induction de l'apoptose, stimulation de la croissance angiogenèse.

6. Procédé selon la revendication 5, dans lequel ledit microorganisme pathogène est spécifique des cellules endothéliales félines.

7. Procédé selon la revendication 5 ou 6, dans lequel ledit microorganisme pathogène est choisi dans le groupe comprenant *Bartonella henselae, Mycoplasma haemofelis, Mycoplasma haemominutum, Anaplasma phagocytophilum et Rickettsia felis..*

8. Procédé in vitro de production de microorganismes pathogènes comprenant les étapes suivantes :
- culture d'au moins une cellule isolée selon la revendication 1 dans un milieu de culture approprié,
- inoculation de ladite cellule cultivée avec ledit microorganisme pathogène,
- la culture des cellules inoculées permettant la prolifération dudit microorganisme pathogène, et
- récupération des microorganismes pathogènes produits.

9. Procédé selon la revendication 8, dans lequel ledit microorganisme pathogène est choisi dans le groupe comprenant *Bartonella henselae, Mycoplasma haemofelis, Mycoplasma haemominutum, Anaplasma phagocytophilum et Rickettsia felis.*

10. Kit de mise en oeuvre du procédé selon la revendication 2 ou 5 comprenant :
- au moins une cellule isolée selon la revendication 1, et
- un moyen de détection de la différentiation et/ou spécialisation cellulaire.

11. Kit de mise en oeuvre du procédé selon la revendication 8 comprenant :
- au moins une cellule isolée selon la revendication 1, et
- un moyen permettant l'inoculation de ladite cellule par un microorganisme pathogène.

12. Culture cellulaire comprenant une cellule isolée selon la revendication 1.

## Patentansprüche

1. Isolierte organospezifische Endothelialzelle der Katze, **dadurch gekennzeichnet, dass** sie den Von-Willebrand-Faktor, das Angiotensin-Converting-Enzym und die Differenzierungscluster (CD) CD 31, 34, 105, 54, 62E, 62P, 146 umfasst, ausgewählt aus den Zellen, hinterlegt in der Collection Nationale de Culture de Microorganismes (CNCM) unter den Nummern CNCM Nr. I-4250 (F Int MEC), Nr. 1-4251 (F PLN MEC), Nr. 1-4252 (FOm EC), Nr. 1-4253 (F Li MEC), Nr. 1-4254 (F Sk MEC), Nr. 1-4255 (F PP MEC), Nr. 1-4256 (F He MEC), Nr. I-4257 (F Br MEC), Nr. 1-4258 (F Lu MEC).

2. Verfahren zum Screenen von Molekülen, die eine Differenzierung und/oder eine Spezialisierung einer isolierten Zelle nach Anspruch 1 induzieren können, umfassend die folgenden Schritte:
- Einführen von mindestens einem Molekül, das gescreent werden soll, in ein Medium, das an die Kultur der Zelle angepasst ist,
- Einführen von mindestens einer isolierten Zelle nach Anspruch 1 in das Medium,
- Kultivieren der Zellen in dem Medium während eines Zeitraums, der ausreichend ist, um die Differenzierung und/oder die Spezialisierung der Zellen zu ermöglichen,
- Entnehmen von mindestens einer kultivierten Zelle aus dem Kulturmedium,
- Beobachten der Differenzierung und/oder Spezialisierung der Zelle durch phänotypische Beobachtung der Zelle und/oder durch Offenbarung von Differenzierungsmarkern.

3. Verfahren nach Anspruch 2, wobei die Offenbarung von Differenzierungsmarkern mit Hilfe von spezifischen Antikörpern der Marker durchgeführt wird.

4. Verfahren nach Anspruch 3, wobei die Moleküle eine Angiogenese induzieren oder hemmen können.

5. Verfahren zur *in* vitro-Untersuchung von Pathologien aufgrund eines pathogenen Mikroorganismus, umfassend die folgenden Schritte:
- Kultivieren von mindestens einer isolierten Zelle nach Anspruch 1 in einem geeigneten Kulturmedium,
- Inokulieren der Zelle mit einem pathogenen Mikroorganismus,
- Kultivieren der inokulierten Zellen, und
- Beobachten von einem oder von mehreren der folgenden Kriterien: Evolution des Phänotyps der Zellen, Evolution der Zytotoxizität, Induktion der Apoptose, Stimulation der Angiogenese.

6. Verfahren nach Anspruch 5, wobei der pathogene Mikroorganismus spezifisch für Endothelialzellen der Katze ist.

7. Verfahren nach Anspruch 5 oder 6, wobei der pathogene Mikroorganismus ausgewählt ist aus der Gruppe umfassend *Bartonella henselae, Mycoplasma haemofelis, Mycoplasma haemominutum, Anaplasma phagocytophilum* und *Rickettsia felis.*

8. *In* vitro-Verfahren zur Produktion von pathogenen Mikroorganismen, umfassend die folgenden Schritte:
- Kultivieren von mindestens einer isolierten Zelle nach Anspruch 1 in einem geeigneten Kulturmedium,
- Inokulieren der kultivierten Zelle mit dem pathogenen Mikroorganismus,
- Kultivieren der inokulierten Zellen, die die Proliferation des pathogenen Mikroorganismus ermöglichen, und
- Rückgewinnen der produzierten pathogenen Mikroorganismen.

9. Verfahren nach Anspruch 8, wobei der pathogene Mikroorganismus ausgewählt ist aus der Gruppe umfassend *Bartonella henselae, Mycoplasma haemofelis, Mycoplasma haemominutum, Anaplasma phagocytophilum* und *Rickettsia felis.*

10. Kit zur Durchführung des Verfahrens nach Anspruch 2 oder 5, umfassend:
- mindestens eine isolierte Zelle nach Anspruch 1, und
- ein Mittel zum Nachweis der Differenzierung und/oder Spezialisierung der Zellen.

11. Kit zur Durchführung des Verfahrens nach Anspruch 8, umfassend:
- mindestens eine isolierte Zelle nach Anspruch 1, und
- ein Mittel, das die Inokulation der Zelle durch einen pathogenen Mikroorganismus ermöglicht.

12. Zellkultur, umfassend eine isolierte Zelle nach Anspruch 1.

## Claims

1. An isolated organ-specific feline endothelial cell, **characterized in that** it comprises the Von Willebrand factor, the angiotensin converting enzyme and the clusters of differentiation (CD) CD 31, 34, 105, 54, 62E, 62P, 146 selected from the cells deposited at the Collection Nationale de Culture de Microorganismes (CNCM), under the CNCM numbers No. I-4250 (F Int MEC), No. I-4251 (F PLN MEC), No. I-4252 (FUm EC), No. I-4253 (F Li MEC), No. I-4254 (F Sk MEC), No. I-4255 (F PP MEC), No. I-4256 (F He MEC), No. I-4257 (F Br MEC), No. I-4258 (F Lu MEC).

2. A method for screening molecules that are able to induce differentiation and/or specialization of an isolated cell according to claim 1 comprising the following steps:
- introducing at least one molecule to be screened into a medium suitable for culture of said cell,
- introducing at least one isolated cell according to claim 1 into said medium,
- culturing said cells in said medium for a sufficient time to permit differentiation and/or specialization of said cells,
- taking at least one cultured cell from said culture medium,
- observing the differentiation and/or specialization of said cell by phenotypic observation of the cell and/or by detecting differentiation markers.

3. The method according to claim 2, wherein the differentiation markers are detected by means of specific antibodies of said markers.

4. The method according to claim 4, wherein said molecules are able to induce, or inhibit angiogenesis.

5. A method for in-vitro investigation of disorders due to a pathogenic microorganism comprising the following steps:
- culturing at least one isolated cell according to claim 1 in a suitable culture medium,
- inoculating said cell with a pathogenic microorganism,
- culturing the inoculated cells, and
- observing one or more of the following criteria: development of the phenotype of the cells, development of cytotoxicity, induction of apoptosis, stimulation of growth of angiogenesis.

6. The method according to claim 5, wherein said pathogenic microorganism is specific to feline endothelial cells.

7. The method according to claim 5 or 6, wherein said pathogenic microorganism is selected from the group comprising *Bartonella henselae, Mycoplasma haemofelis, Mycoplasma haemominutum, Anaplasma phagocytophilum* and *Rickettsia felis.*

8. An in-vitro method for producing pathogenic microorganisms comprising the following steps:
- culturing at least one isolated cell according to claim 1 in a suitable culture medium,
- inoculating said cultured cell with said pathogenic microorganism,
- culturing the inoculated cells permitting proliferation of said pathogenic microorganism, and
- recovery of the pathogenic microorganisms produced.

9. The method according to claim 8, wherein said pathogenic microorganism is selected from the group comprising *Bartonella henselae, Mycoplasma haemofelis, Mycoplasma haemominutum, Anaplasma phagocytophilum* and *Rickettsia felis.*

10. A kit for carrying out the method according to claim 2 or 5 comprising:
- at least one isolated cell according to claim 1, and
- a means for detecting cellular differentiation and/or specialization.

11. A kit for carrying out the method according to claim 8 comprising:
- at least one isolated cell according to claim 1, and
- a means for inoculating said cell with a pathogenic microorganism.

12. A cell culture comprising an isolated cell according to claim 1.
